# EUROPEAN PATENT APPLICATION

(11) **EP 4 786 959 A1**
(43) Date of publication of application: **05.08.2026**
(21) Application number: 25192579.8
(22) Date of filing: 29.07.2025
(51) Int. Cl.: G01N 21/86, G03G 15/00, G01N 33/34

(54) **INFORMATION PROCESSING SYSTEM**

(30) Priority: 30.01.2025 JP 2025013981
(71) Applicant: FUJIFILM Business Innovation Corp., Minato-ku Tokyo (JP)
(72) Inventor: TANAKA, Kumiko, Ebina-shi, Kanagawa (JP); OMORI, Masao, Ebina-shi, Kanagawa (JP)
(74) Representative: Becker Kurig & Partner Patentanwälte mbB

(57) **Abstract**

An information processing system is disposed in an image forming apparatus (1), such as a printer, and includes: a processor (21b) configured to: check a fiber orientation indicating an orientation state of fibers in paper (10) from plural directions; and derive an expansion and contraction ratio of the paper (10) based on the checked fiber orientation.

## Description

### BACKGROUND OF THE INVENTION

### (i) Field of the Invention

The present invention relates to an information processing system.

### (ii) Description of Related Art

For example, JP2010-250017A discloses a configuration in which an irradiation LED, a CMOS area sensor that images a light irradiation region as a surface image, and a drive and calculation portion that detects information on a roughness state of a surface of a recording material are provided, the irradiation LED is disposed to have a relationship such that a straight line connecting the irradiation LED and the light irradiation region, which is obtained by projecting the straight line onto the recording material and a straight line connecting the irradiation LED and the light irradiation region, which is obtained by projecting the straight line onto the recording material intersect with each other, and in a case of imaging the light irradiation region as the surface image, times at which each of the irradiation LEDs is turned on and off are different, and a surface state of the recording material is detected based on the surface image obtained at each time.

### SUMMARY OF THE INVENTION

Here, since an expansion and contraction ratio of paper affects, for example, a print quality, it is preferable to grasp the expansion and contraction ratio of the paper before printing and control, for example, a printing condition based on the grasped expansion and contraction ratio.

Meanwhile, in a case where the expansion and contraction ratio of the paper is measured by using a device in the related art, it takes time to process the paper to a predetermined size and the like, and it takes a long time to evaluate the expansion and contraction ratio. Therefore, it takes a long time to grasp the expansion and contraction ratio.

An object of the present invention is to shorten a time until an expansion and contraction ratio is grasped, as compared with a case where the expansion and contraction ratio of paper is measured by using a device in the related art.

According to a first aspect of the present invention, there is provided an information processing system including: a processor configured to: check a fiber orientation indicating an orientation state of fibers in paper from a plurality of directions; and derive an expansion and contraction ratio of the paper based on the checked fiber orientation.

According to a second aspect of the present invention, in the information processing system according to the first aspect, the checking of the fiber orientation may be performed by using a first image in a case where a surface of the paper is irradiated with light from one direction as the plurality of directions and a second image in a case where the surface of the paper is irradiated with light from another direction as the plurality of directions.

According to a third aspect of the present invention, in the information processing system according to the second aspect, the irradiation with the light from the one direction may be performed at a first angle with respect to the other direction, and the irradiation with the light from the other direction may be performed at a second angle with respect to the one direction.

According to a fourth aspect of the present invention, in the information processing system according to the third aspect, the first angle may be any angle in a range determined based on an error with respect to the expansion and contraction ratio corresponding to an angle with respect to the other direction, and the second angle may be any angle in a range determined based on an error with respect to the expansion and contraction ratio corresponding to an angle with respect to the one direction.

According to a fifth aspect of the present invention, in the information processing system according to the third aspect, the first angle and the second angle may be any angles in a range of 70 degrees to 90 degrees.

According to a sixth aspect of the present invention, in the information processing system according to the second aspect, the irradiation with the light from the one direction may be performed by reducing an intensity of light at an irradiation position by the light from the other direction relative to an intensity of light at the irradiation position by the light from the one direction, and the irradiation with the light from the other direction may be performed by reducing the intensity of light at the irradiation position by the light from the one direction relative to the intensity of light at the irradiation position by the light from the other direction.

According to a seventh aspect of the present invention, in the information processing system according to the sixth aspect, the reducing of the intensity of light at the irradiation position by the light from the other direction may be realized by reducing a luminous intensity of the light from the other direction, and the reducing of the intensity of light at the irradiation position by the light from the one direction may be realized by reducing a luminous intensity of the light from the one direction.

According to an eighth aspect of the present invention, in the information processing system according to the sixth aspect, the reducing of the intensity of light at the irradiation position by the light from the other direction may be realized by restricting the light from the other direction by one member, and the reducing of the intensity of light at the irradiation position by the light from the one direction may be realized by restricting the light from the one direction by another member.

According to a ninth aspect of the present invention, in the information processing system according to the second aspect, the deriving of the expansion and contraction ratio may be performed by a combination of any two or more of a first fiber orientation checked by the first image, a second fiber orientation checked by the second image, or a difference between the first fiber orientation and the second fiber orientation.

According to a tenth aspect of the present invention, in the information processing system according to any one of the first aspect to the eighth aspect, a fiber density of the paper may be checked from a direction different from the plurality of directions, and the deriving of the expansion and contraction ratio may be performed based on the fiber orientation and the fiber density.

According to an eleventh aspect of the present invention, in the information processing system according to the tenth aspect, the direction different from the plurality of directions may be a direction in which light is emitted to an irradiation position of one surface of the paper irradiated with light from one direction and light from another direction, from another surface of the paper, and the checking of the fiber density may be performed by using an image of the light with which the other surface is irradiated and which is transmitted through the paper.

According to a twelfth aspect of the present invention, in the information processing system according to the eleventh aspect, the deriving of the expansion and contraction ratio may be performed by a combination including the fiber density, and including at least one of a first fiber orientation checked by a first image, a second fiber orientation checked by a second image, or a difference between the first fiber orientation and the second fiber orientation.

According to a thirteenth aspect of the present invention, in the information processing system according to any one of the first aspect to the twelfth aspect, a temperature at which an image formed on the paper is to be fixed may be controlled based on the derived expansion and contraction ratio.

According to a fourteenth aspect of the present invention, in the information processing system according to the thirteenth aspect, in a case where the expansion and contraction ratio is equal to or higher than a predetermined value, the fixing temperature may be set to be lower than a predetermined temperature.

According to a fifteenth aspect of the present invention, in the information processing system according to the thirteenth aspect, in a case where the expansion and contraction ratio is equal to or lower than a predetermined value, the fixing temperature may be set to be higher than a predetermined temperature.

According to the first aspect of the present invention, it is possible to shorten a time taken to grasp the expansion and contraction ratio, as compared with a case where the expansion and contraction ratio of the paper is measured by using a device in the related art.

According to the second aspect of the present invention, a burden of the checking process can be reduced, as compared with a case where the configuration is not adopted in which the checking of the fiber orientation is performed by using the first image in a case where the surface of the paper is irradiated with light in the one direction of the plurality of directions and the second image in a case where the surface of the paper is irradiated with light in the other direction of the plurality of directions.

According to the third aspect of the present invention, accuracy of the expansion and contraction ratio can be improved, as compared with a case where the configuration in which the irradiation of light from the one direction is performed at the first angle with respect to the other direction and the irradiation of light from the other direction is performed at the second angle with respect to the one direction is not adopted.

According to the fourth aspect of the present invention, an angle according to the paper can be set, as compared with a case where the configuration is not adopted in which the first angle is any angle in a range determined based on the error with respect to the expansion and contraction ratio corresponding to an angle with respect to the other direction, and the second angle is any angle in a range determined based on the error with respect to the expansion and contraction ratio corresponding to an angle with respect to the one direction.

According to the fifth aspect of the present invention, accuracy of the expansion and contraction ratio can be improved, as compared with a case where the first angle and the second angle are not any angle within a range of 70 degrees to 90 degrees.

According to the sixth aspect of the present invention, it is possible to suppress blurriness of a shadow, as compared with a case where the configuration is not adopted in which the irradiation of the light from the one direction is performed by reducing the intensity of light at the irradiation position by the light from the other direction than the intensity of light at the irradiation position by the light from the one direction, and the irradiation of the light from the other direction is performed by reducing the intensity of light at the irradiation position by the light from the one direction than the intensity of light at the irradiation position by the light from the other direction.

According to the seventh aspect of the present invention, a fluctuation in the luminous intensity of the light during the lighting can be suppressed, as compared with a case where the configuration is not adopted in which the reducing of the intensity of light at the irradiation position by the light from the other direction is realized by reducing the luminous intensity of the light from the other direction, and the reducing of the intensity of light at the irradiation position by the light from the one direction is realized by reducing the luminous intensity of the light from the one direction.

According to the eighth aspect of the present invention, it is possible to reduce a control burden, as compared with a case where the configuration is not adopted in which the reducing of the intensity of light at the irradiation position by the light from the other direction is realized by restricting the light from the other direction by the one member, and the reducing of the intensity of light at the irradiation position by the light from the one direction is realized by restricting the light from the one direction by the other member.

According to the ninth aspect of the present invention, an error of the expansion and contraction ratio can be suppressed, as compared with a case where the configuration is not adopted in which the expansion and contraction ratio is derived by a combination of any two or more of the first fiber orientation checked by the first image, the second fiber orientation checked by the second image, and the difference between the first fiber orientation and the second fiber orientation.

According to the tenth aspect of the present invention, the accuracy of the expansion and contraction ratio can be improved as compared with a case where the configuration is not adopted in which the fiber density of the paper is checked from a direction different from the plurality of directions, and the expansion and contraction ratio is derived based on the fiber orientation and the fiber density.

According to the eleventh aspect of the present invention, the accuracy of the expansion and contraction ratio can be improved, as compared with a case where the configuration is not adopted in which the direction different from the plurality of directions is a direction in which light is emitted to the irradiation position of one surface of the paper irradiated with light from one direction and light from another direction, from another surface of the paper, and the checking of the fiber density is performed by using an image of the light with which the other surface is irradiated and which is transmitted through the paper.

According to the twelfth aspect of the present invention, the error of the expansion and contraction ratio can be suppressed, as compared with a case where the configuration is not adopted in which the deriving of the expansion and contraction ratio is performed by a combination including the fiber density, and including at least one of the first fiber orientation checked by the first image, the second fiber orientation checked by the second image, or the difference between the first fiber orientation and the second fiber orientation.

According to the thirteenth aspect of the present invention, it is possible to suppress occurrence of curling of the paper, as compared to a case where the configuration is not adopted in which the temperature at which the image formed on the paper is to be fixed is controlled based on the derived expansion and contraction ratio.

According to the fourteenth aspect of the present invention, the occurrence of curling of the paper can be suppressed, as compared with a case where the configuration is not adopted in which in a case where the expansion and contraction ratio is equal to or higher than the predetermined value, the fixing temperature is set to be lower than the predetermined temperature.

According to the fifteenth aspect of the present invention, the occurrence of curling of the paper can be suppressed, as compared with a case where the configuration is not adopted in which in a case where the expansion and contraction ratio is equal to or lower than the predetermined value, the fixing temperature is set to be higher than the predetermined temperature.

### BRIEF DESCRIPTION OF THE DRAWINGS

Exemplary embodiment(s) of the present invention will be described in detail based on the following figures, wherein:
FIG. 1 is a diagram describing an image forming apparatus;
FIG. 2 is a diagram illustrating an example of a hardware configuration of a control portion of the image forming apparatus;
FIG. 3 is a diagram describing a configuration for measuring an expansion and contraction ratio provided in the image forming apparatus;
FIGS. 4A and 4B are diagrams describing a relationship between a light source and paper, in which FIG. 4A is a plan view illustrating a positional relationship between light sources 7a and 7b and paper, and FIG. 4B is a front view illustrating a positional relationship between a light source 7c and paper;
FIG. 5 is a front view schematically illustrating a relationship between the light source, a light detector, and paper;
FIG. 6 is a graph describing a relationship between an irradiation angle of the light source and a predicted expansion and contraction ratio, in which a vertical axis represents the predicted expansion and contraction ratio (%), and a horizontal axis represents the irradiation angle of the light source (degree);
FIG. 7 is a flowchart illustrating an example of a processing procedure in a case of capturing an image of a portion with light from the light source;
FIGS. 8A and 8B are diagrams describing a configuration example of a shutter member, in which FIG. 8A illustrates a case where light from the light source is not shielded by the shutter member, and FIG. 8B illustrates a case where the light from the light source is shielded by the shutter member;
FIG. 9 is a histogram describing a process of an image acquisition portion of an information processing portion, in which a vertical axis is a frequency (the number of pixels) and a horizontal axis is a class of luminance (cd/m²);
FIGS. 10A and 10B are graphs illustrating a fiber orientation of an image by light reflected by paper, in which FIG. 10A illustrates an image using the light source 7a and FIG. 10B illustrates an image using the light source 7b;
FIG. 11 is a diagram illustrating a fiber density in a thickness direction of paper;
FIGS. 12A and 12B are graphs illustrating a fiber density of an image by light transmitted through paper, in which FIGS. 12A and 12B illustrate a case of the image by the light source 7c;
FIG. 13 is a cross-sectional view illustrating a state of roughness on one surface of paper;
FIGS. 14A and 14B are diagrams describing prediction accuracy in a case of predicting an expansion and contraction ratio in a cross grain direction, in which FIG. 14A is a table illustrating an extraction result and FIG. 14B is a matrix diagram illustrating a correlation between a predictive value of the expansion and contraction ratio and an actual measurement value of the expansion and contraction ratio, respectively;
FIG. 15 is a diagram describing prediction accuracy in a case of predicting the expansion and contraction ratio in the cross grain direction;
FIGS. 16A and 16B are diagrams describing prediction accuracy in a case of predicting the expansion and contraction ratio in the cross grain direction, in which FIG. 16A is a table illustrating an extraction result and FIG. 16B is a matrix diagram illustrating a correlation between a predictive value of the expansion and contraction ratio and an actual measurement value of the expansion and contraction ratio, respectively;
FIG. 17 is a flowchart illustrating a processing example of correcting a size of an image formed on paper according to the expansion and contraction ratio of the paper;
FIGS. 18A and 18B are tables describing a first specific example of calculation of a correction value, in which FIG. 18A is a case of printing on a front surface and FIG. 18B is a case of printing on a back surface;
FIG. 19 is a table describing a second specific example of the calculation of the correction value;
FIG. 20 is a diagram illustrating a curl state of paper in a fixing device;
FIG. 21 is a graph describing an occurrence state of a peeling type jam in a case where paper is heated, in which each vertical axis represents a CD expansion and contraction ratio and each horizontal axis represents a CD bending stiffness;
FIG. 22 is a graph describing an occurrence state of another peeling type jam in a case where the paper is heated, in which each vertical axis represents a CD expansion and contraction ratio and each horizontal axis represents a CD bending stiffness;
FIG. 23 is a graph describing an occurrence state of still another peeling type jam in a case where the paper is heated, in which each vertical axis represents a CD expansion and contraction ratio and each horizontal axis represents a CD bending stiffness;
FIGS. 24A and 24B are diagrams describing prediction accuracy in a case of predicting an expansion and contraction ratio in the cross grain direction of recycled paper, in which FIG. 24A is a table illustrating an extraction result and FIG. 24B is a matrix diagram illustrating a correlation between a predictive value of the expansion and contraction ratio and an actual measurement value of the expansion and contraction ratio, respectively; and
FIGS. 25A and 25B are diagrams describing the prediction accuracy in a case of predicting the expansion and contraction ratio in the cross grain direction of recycled paper, in which FIG. 25A is a table illustrating an extraction result and FIG. 25B is a matrix diagram illustrating a correlation between a predictive value of the expansion and contraction ratio and an actual measurement value of the expansion and contraction ratio, respectively.

### DETAILED DESCRIPTION OF THE INVENTION

Hereinafter, exemplary embodiments of the present invention will be described in detail with reference to the accompanying drawings.

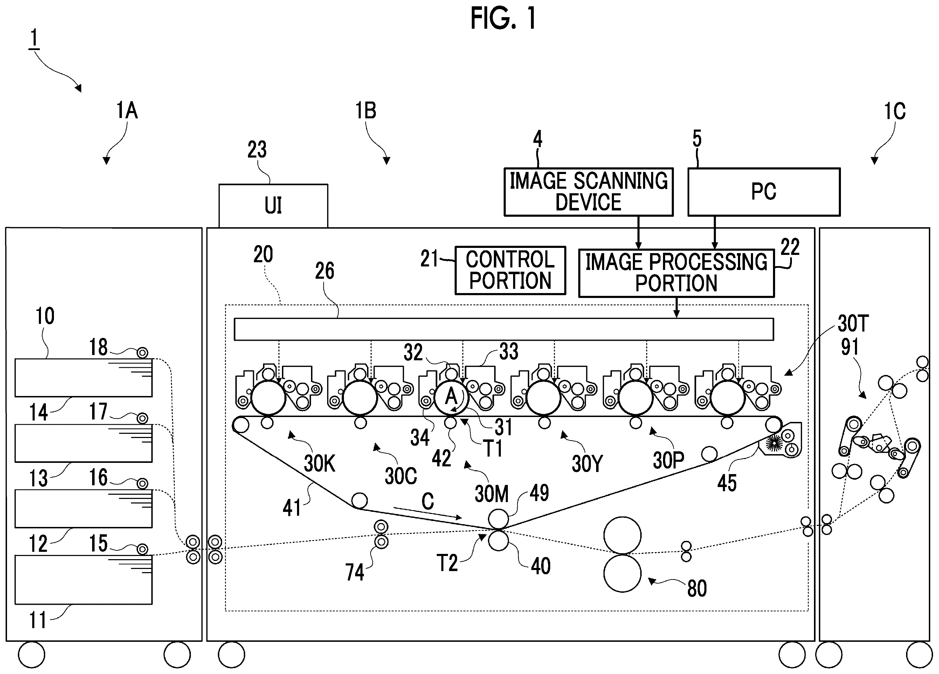

FIG. 1 is a diagram describing an image forming apparatus 1.

The image forming apparatus 1 according to the present exemplary embodiment includes a paper feeding unit 1A, a printing unit 1B, and a paper discharge unit 1C.

The paper feeding unit 1A includes a first paper accommodation portion 11 to a fourth paper accommodation portion 14 that accommodate paper 10 as an example of a recording medium.

In addition, feeding rolls 15 to 18 that are provided to correspond to the first paper accommodation portion 11 to the fourth paper accommodation portion 14, respectively, and that feed the paper 10 accommodated in each paper accommodation portion to a transport path connected to the printing unit 1B are provided in the paper feeding unit 1A.

The printing unit 1B includes an image forming portion 20 that forms an image on the paper 10. In addition, a control portion 21 that controls each portion of the image forming apparatus 1 is provided in the printing unit 1B.

In addition, the printing unit 1B includes an image processing portion 22. The image processing portion 22 performs an image process on image data transmitted from an image scanning device 4 or a personal computer (PC) 5.

In addition, a user interface (UI) 23 that is configured with a touch panel or the like and that notifies a user of information and receives an input of information from the user is provided in the printing unit 1B.

Six image forming units 30T, 30P, 30Y, 30M, 30C, and 30K (hereinafter, may be simply referred to as "image forming units 30") disposed parallel to each other at constant intervals are provided in the image forming portion 20.

Each image forming unit 30 includes a photosensitive drum 31 on which an electrostatic latent image is formed while rotating in a direction of an arrow A and a charging roll 32 that charges a surface of the photosensitive drum 31. In addition, each image forming unit 30 includes a developer 33 that develops the electrostatic latent image formed on the photosensitive drum 31 and a drum cleaner 34 that removes toner and the like on the surface of the photosensitive drum 31.

In addition, the image forming portion 20 is provided with a laser exposure device 26 that exposes each photosensitive drum 31 of each image forming unit 30 with laser light.

The exposure of the photosensitive drum 31 by the laser exposure device 26 is not limited to using laser light. For example, each image forming unit 30 may be provided with a light source such as a light emitting diode (LED), and the exposure of the photosensitive drum 31 may be performed by using light emitted from the light source.

Each image forming unit 30 has the same configuration, except for toner accommodated in the developer 33. The image forming units 30Y, 30M, 30C, and 30K form yellow (Y), magenta (M), cyan (C), and black (K) toner images, respectively.

In addition, the image forming units 30T and 30P form toner images using toner corresponding to corporate colors, foamed toner for braille, fluorescent toner, toner to improve glossiness, and the like. In other words, the image forming units 30T and 30P form toner images using special color toner.

In addition, the image forming portion 20 is provided with an intermediate transfer belt 41 to which the toner image of each color formed on the photosensitive drum 31 of each image forming unit 30 is transferred.

In addition, the image forming portion 20 is provided with a primary transfer roll 42 that transfers each color toner image of each image forming unit 30 onto the intermediate transfer belt 41 at a primary transfer portion T1.

In addition, the image forming portion 20 is provided with a secondary transfer roll 40 that transfers the toner images transferred onto the intermediate transfer belt 41 at once onto the paper 10 at a secondary transfer portion T2.

Further, the image forming portion 20 is provided with a belt cleaner 45 that removes toner and the like on a surface of the intermediate transfer belt 41, and a fixing device 80 that fixes the secondarily transferred image onto the paper 10.

The image forming portion 20 performs an image forming operation based on a control signal from the control portion 21.

Specifically, in the image forming portion 20, first, an image process is performed by the image processing portion 22 on the image data input from the image scanning device 4 or the PC 5, and the image data after the image process is performed is supplied to the laser exposure device 26.

Then, for example, in the image forming unit 30M for magenta (M), after the surface of the photosensitive drum 31 is charged by the charging roll 32, the photosensitive drum 31 is irradiated by the laser exposure device 26 with laser light modulated with the image data obtained from the image processing portion 22.

Therefore, the electrostatic latent image is formed on the photosensitive drum 31.

The formed electrostatic latent image is developed by the developer 33, and a magenta toner image is formed on the photosensitive drum 31.

Similarly, in the image forming units 30Y, 30C, and 30K, yellow, cyan, and black toner images are formed, and in the image forming units 30T and 30P, special color toner images are formed.

Each color toner image formed in each image forming unit 30 is sequentially electrostatically transferred to the intermediate transfer belt 41 rotating in a direction of an arrow C in FIG. 1 by the primary transfer roll 42, and a superimposed toner image is formed on the intermediate transfer belt 41.

The superimposed toner image formed on the intermediate transfer belt 41 is transported to the secondary transfer portion T2 that is configured with the secondary transfer roll 40 and a backup roll 49, as the intermediate transfer belt 41 is moved.

Meanwhile, for example, the paper 10 is taken from the first paper accommodation portion 11 by the feeding roll 15, and is then transported to a position of a registration roll 74 through the transport path.

In a case where the superimposed toner image is transported to the secondary transfer portion T2, the paper 10 is supplied from the registration roll 74 to the secondary transfer portion T2 in accordance with a time of the transportation.

Then, at the secondary transfer portion T2, the superimposed toner image is electrostatically transferred at once onto the paper 10 by an action of a transfer electric field formed between the secondary transfer roll 40 and the backup roll 49.

Then, the paper 10 on which the superimposed toner image is electrostatically transferred is transported to the fixing device 80.

In the fixing device 80, the paper 10 on which the unfixed toner image is formed is pressurized and heated, and a fixing process of the toner image on the paper 10 is performed.

The paper 10 on which the fixing process is performed passes through a curl correction portion 91 provided in the paper discharge unit 1C and is then transported to a paper stacking portion (not illustrated).

### <Control Portion 21>

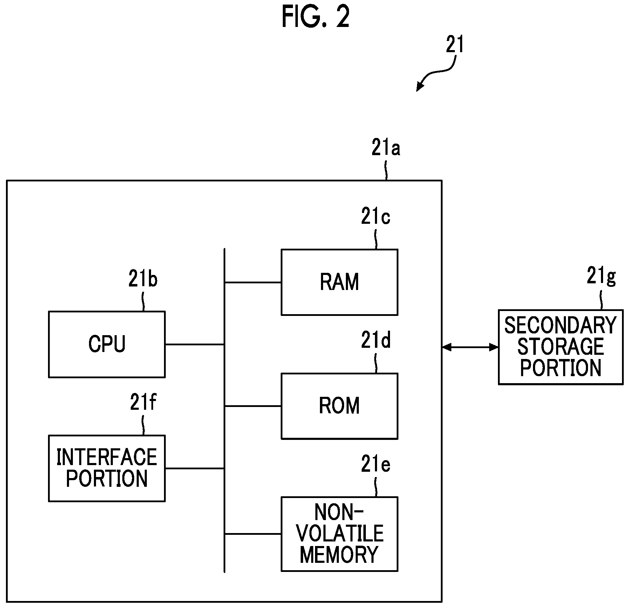

FIG. 2 is a diagram illustrating an example of a hardware configuration of the control portion 21 of the image forming apparatus 1. The control portion 21 is realized by a computer.

The control portion 21 includes an arithmetic processing portion 21a that executes a digital arithmetic process according to a program, and a secondary storage portion 21g that stores information.

The secondary storage portion 21g is realized, for example, by a known information storage device such as a hard disk drive (HDD), a semiconductor memory, or a magnetic tape.

A CPU 21b as an example of a processor is provided in the arithmetic processing portion 21a.

In addition, the arithmetic processing portion 21a is provided with a RAM 21c used as a work memory or the like of the CPU 21b and a ROM 21d in which a program executed by the CPU 21b and the like are stored.

In addition, the arithmetic processing portion 21a is provided with a non-volatile memory 21e that is configured to be rewritable and can store data even in a case where power supply is interrupted and an interface portion 21f that controls each portion, such as a communication unit, connected to the arithmetic processing portion 21a.

The non-volatile memory 21e is configured with, for example, an SRAM, a flash memory, or the like that is backed up by a battery. The secondary storage portion 21g stores the program executed by the arithmetic processing portion 21a, in addition to a file and the like.

In the present exemplary embodiment, the arithmetic processing portion 21a reads the program stored in the ROM 21d or the secondary storage portion 21g to execute each process.

Here, the program executed by the CPU 21b may be provided to the image forming apparatus 1 in a state of being stored in a computer-readable recording medium such as a magnetic recording medium (such as a magnetic tape or a magnetic disk), an optical recording medium (such as an optical disk), a magneto-optical recording medium, or a semiconductor memory. In addition, the program executed by the CPU 21b may be provided to the image forming apparatus 1 by using communication means such as the Internet.

In the exemplary embodiments, the processes are performed by any computer. The computer may perform the processes by using a processor serving as hardware, a program serving as software, or combination of these. In this case, the processor is configured to perform the processes in the exemplary embodiments in cooperation with the program and may function as a unit or a means in the exemplary embodiments. The order in which the processor performs the processes is not limited to the described order and may be changed appropriately. The computer may be a general-purpose computer, an application specific computer, a workstation, or another system capable of performing the processes.

The processor may be composed of one or more pieces of hardware, and the type of the hardware is not limited. For example, the processor may be composed of hardware such as a central processing unit (CPU), a micro processing unit (MPU), a programmable logic device such as a field programmable gate array (FPGA), a dedicated circuit for performing specific processing such as an application specific integrated circuit (ASIC), a graphics processing unit (GPU), or a neural processing unit (NPU). Regarding the type of the hardware, different types of hardware may be combined. If multiple pieces of hardware are configured to perform one or more processes of the processor, the multiple pieces of hardware may be present in apparatuses physically away from each other or may be present in one apparatus. In each of exemplary embodiments, the order in which the processor performs the processes is not limited to the order described above and may be changed appropriately. The hardware is composed of electric circuitry in which circuit elements such as semiconductor devices are combined, or the like.

Further, the program may be software such as firmware or microcode. The program may be, for example, a program module group, and the functions thereof may be implemented by processors configured to implement the respective functions. The program may be program code or multiple code segments stored in one or more non-transitory computer readable media (for example, a storage medium or another storage). The program may be stored in such a divided manner in multiple non-transitory computer readable media present in apparatuses physically away from each other. The program code or the code segments may represent a procedure, a function, a sub program, a routine, a subroutine, a module, a software package, a class or any combination of instructions, data structures, or program statements. The program code or the code segment may be connected to another code segment or a hardware circuit by transmitting and/or receiving information, data, an argument, a parameter, or memory content.

Here, an expansion and contraction ratio of the paper 10 in the image forming apparatus 1 varies depending on a manufacturing lot variation (factory difference, seasonal difference) of the paper 10 or a use environment, and affects a print quality such as curling of the paper 10 during transportation or fixing. In a case where the expansion and contraction ratio of the paper 10 is accurately grasped immediately before an image is formed on the paper 10 and the grasped expansion and contraction ratio of the paper 10 is reflected in the control of the printing condition or the like, it is possible to suppress the influence on the print quality.

Meanwhile, an expansion and contraction ratio measuring device in the related art is large and requires the paper 10 to be processed into a predetermined size, and moreover, it takes a long time for evaluation.

Therefore, in the present exemplary embodiment, an expansion and contraction ratio measurement model for shortening a time until the expansion and contraction ratio of the paper 10 is grasped in the image forming apparatus 1 is provided. Hereinafter, description will be made.

Since the paper 10 is made of a plant fiber as a major raw material, the paper 10 expands and contracts due to an increase or decrease in moisture. The plant fiber expands by moisture absorption and contracts by dehumidification, and the degree of expansion and the contraction is large in a lateral direction (diameter) of a single fiber and is small in a longitudinal direction (length) of the single fiber.

In the paper 10, the contraction of the entire paper 10 occurs due to the expansion and contraction of the individual fibers caused by a change in humidity over the entire surface through the inter-fiber bonds.

During paper making, the fibers tend to be arranged in a flow direction. Therefore, the expansion and contraction due to a change in humidity is larger in a direction (lateral direction) perpendicular to the machine flow direction (longitudinal direction). As a test method, an inside of a device is set to various relative humidities, moisture of a test piece is changed, the amount of expansion and contraction with respect to the original size at a reference humidity is measured, and the result is expressed as an expansion and contraction ratio % (hygroexpansivity).

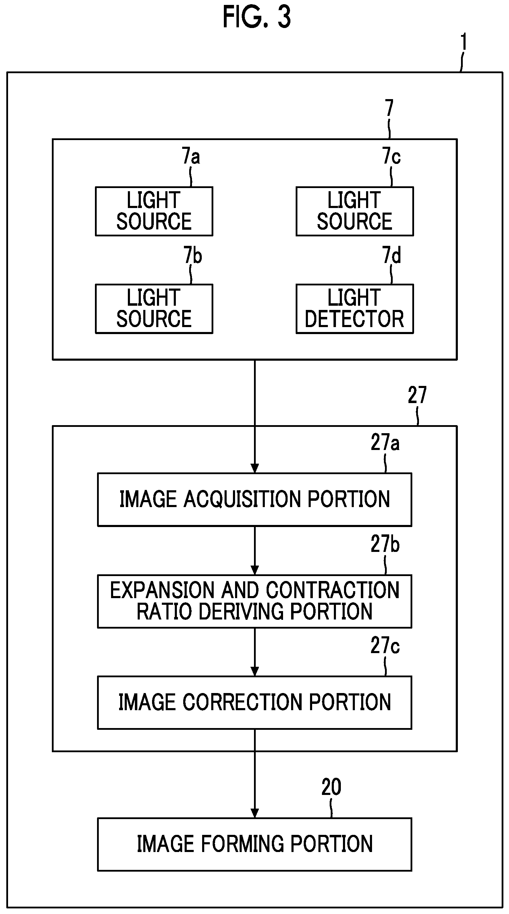

FIG. 3 is a diagram describing a configuration for measuring an expansion and contraction ratio provided in the image forming apparatus 1.

The image forming apparatus 1 includes a media sensor 7 and an information processing portion 27.

The media sensor 7 is disposed at any position on a paper transport path in the image forming portion 20 such that light can be emitted to the transported paper 10 and light transmitted through the paper 10 can be detected. More specifically, in a case of an electrophotographic method, the media sensor 7 is disposed at any position up to the secondary transfer portion T2 (see FIG. 1) of the paper transport path. **In** addition, in a case of the image forming portion 20 having a two-sided printing function, it is conceivable to provide the media sensor 7 in a transport path through which the paper 10 passes during both front surface printing and back surface printing. Therefore, the measurement of the paper 10 immediately before the front surface printing and immediately before the back surface printing can be performed by one media sensor 7.

The media sensor 7 is a device for detecting one or more characteristics of the paper 10.

The characteristic detected by the media sensor 7 includes an absorbance of light in a wavelength band of interest. In order to detect the absorbance, the media sensor 7 includes light sources 7a, 7b, and 7c that project light in a wavelength band including a wavelength band of interest toward the paper 10. In addition, the media sensor 7 includes a light detector 7d that detects light emitted from the light sources 7a, 7b, and 7c and reflected by or transmitted through the paper 10.

As the light sources 7a to 7c, for example, LEDs can be used. In addition, it is considered that, for example, a camera is used as the light detector 7d. More specifically, it is considered that the light sources 7a to 7c are white LEDs and the light detector 7d is a CMOS camera.

In the present exemplary embodiment, the media sensor 7 includes three LEDs as the light sources 7a to 7c and one camera as the light detector 7d.

More specifically, the light from the light sources 7a and 7b is reflected by the paper 10 and is detected by the light detector 7d. The light sources 7a and 7b are disposed to emit light in directions different from each other with respect to the paper 10.

The light from the light source 7c is transmitted through the paper 10 and is detected by the light detector 7d.

The light detector 7d has at least a function of extracting and detecting light in the wavelength band of interest. The light detector 7d may have a function of detecting one or more characteristics other than the absorbance of light in the wavelength band of interest, for example, a thickness of the paper 10, a smoothness of the surface, a basis weight, and the like. The media sensor 7 includes one or more sensors corresponding to one or more characteristics to be detected.

In the present exemplary embodiment, a configuration in which the media sensor 7 includes one camera is adopted, so that the configuration of the media sensor 7 is simplified. In addition, with such a configuration, in the present exemplary embodiment, a size of the media sensor 7 is reduced. As a modification example thereof, a configuration in which the media sensor 7 includes a plurality of cameras is considered.

In addition, in the present exemplary embodiment, a configuration in which the light sources 7a and 7b are disposed as two fixed light sources is adopted. As a modification example thereof, a configuration in which one light source that can be moved with respect to the paper 10 to be irradiated is provided instead of the two fixed light sources is considered. That is, this is a configuration example in which one light source is rotated with respect to the paper 10 and an image is captured by a camera. More specifically, a configuration example is adopted in which one movable light source can be displaced to a position of the light source 7a and a position of the light source 7b to reduce the number of light sources. In such a modification example, imaging may be performed at a position other than the position of the light source 7a and the position of the light source 7b.

The information processing portion 27 includes an image acquisition portion 27a, an expansion and contraction ratio deriving portion 27b, and an image correction portion 27c. The information processing portion 27 is configured with the control portion 21 (for example, see FIG. 1).

The image acquisition portion 27a acquires an image in a case where light is applied from each of a plurality of directions, as a detection result of the light detector 7d of the media sensor 7. In addition, the image acquisition portion 27a checks a fiber orientation of the paper 10 from the plurality of directions by using a plurality of acquired images. That is, it is checked how many fibers are included in the paper 10 in a grain direction to be described below or a cross grain direction to be described below, and which direction the fibers are oriented.

The expansion and contraction ratio deriving portion 27b derives an expansion and contraction ratio of the paper 10 based on the fiber orientation checked by the image acquisition portion 27a. The derivation of the expansion and contraction ratio described herein refers to predicting the expansion and contraction ratio.

The expansion and contraction ratio deriving portion 27b derives the expansion and contraction ratio in a plurality of directions of the paper 10. That is, the expansion and contraction ratio deriving portion 27b derives the expansion and contraction ratio in a direction of one side 10c and the expansion and contraction ratio in a direction of another side 10d.

The image correction portion 27c corrects an image to be formed on the paper 10 according to the expansion and contraction ratio of the paper 10 derived by the expansion and contraction ratio deriving portion 27b. In this manner, the information processing portion 27 derives the expansion and contraction ratio of the paper 10 according to measurement of the media sensor 7, and performs, for example, control of enlarging or reducing the image to be formed on the paper 10 according to the expansion and contraction ratio.

Image data subjected to such correction is supplied to the image forming portion 20 and is printed on the paper 10.

In the present exemplary embodiment, a configuration in which the control portion 21 (for example, see FIG. 1) includes the image correction portion 27c is adopted, but the present invention is not limited thereto, and a configuration in which the image processing portion 22 (see FIG. 1) includes the image correction portion 27c is also considered. In addition, a control example in which information indicating the expansion and contraction ratio derived by the expansion and contraction ratio deriving portion 27b is stored in the RAM 21c (see FIG. 2) and is read out as necessary is considered.

### <Media Sensor 7>

Next, a relationship between the media sensor 7 and the paper 10 will be described. As described above, the media sensor 7 is configured to include the light sources 7a to 7c and the light detector 7d (see FIG. 3).

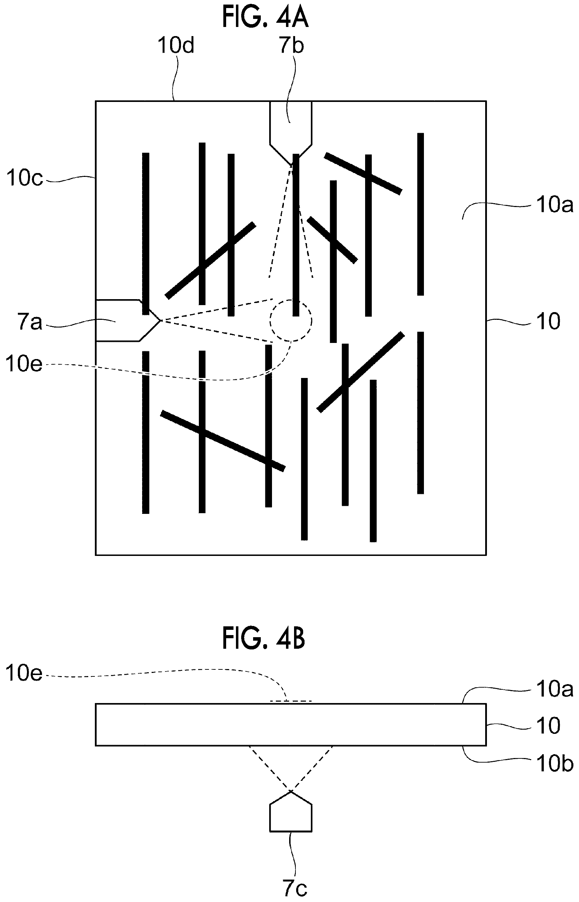

FIGS. 4A and 4B are diagrams describing a relationship between the light sources 7a to 7c and the paper 10, in which FIG. 4A is a plan view illustrating a positional relationship between the light sources 7a and 7b and the paper 10, and FIG. 4B is a front view illustrating a positional relationship between the light source 7c and the paper 10.

Any one of a front surface or a back surface of the paper 10 is referred to as one surface 10a, and the other is referred to as another surface 10b. In this manner, one surface 10a and the other surface 10b are used without specifying whether the surface is the front surface or the back surface of the paper 10, and without specifying whether the surface is the upper surface or the lower surface of the paper 10. Meanwhile, for example, the front surface of the paper 10 may be specified as one surface 10a, and the back surface may be specified as the other surface 10b. In such a case, one surface 10a is an example of the front surface of the paper, and the other surface 10b is an example of the back surface of the paper.

In addition, any one of a long side or a short side of the quadrangular paper 10 is referred to as one side 10c, and the other side is referred to as the other side 10d.

As illustrated in FIG. 4A, both the light sources 7a and 7b are disposed on one surface 10a side of the paper 10 and irradiate one surface 10a.

More specifically, the light source 7a is disposed on one side 10c and emits light in a direction in which the other side 10d extends. In addition, the light source 7b is disposed on the other side 10d and emits light in a direction in which one side 10c extends.

More specifically, the light source 7a emits light toward a portion 10e of the paper 10. In addition, the light source 7b emits light toward the portion 10e irradiated by the light source 7a. That is, the portion 10e of the paper 10 is a common region in which the light of the light source 7a is irradiated and the light of the light source 7b is also irradiated. The portion 10e is irradiated with the light of the light sources 7a and 7b in order. That is, the irradiation of the light sources 7a and 7b is performed on the portion 10e with a time difference. Any of the irradiations may be performed first.

The light sources 7a and 7b are not parallel to each other in a direction in which light is emitted, but intersect each other.

As illustrated in FIG. 4B, the light source 7c is disposed on the other surface 10b side of the paper 10 and irradiates the other surface 10b. That is, the light source 7c is disposed on a surface of the paper 10 opposite to the light sources 7a and 7b.

More specifically, the light source 7c is disposed at a position corresponding to the portion 10e of the paper 10, and emits light toward the portion 10e. That is, the light of the light source 7c irradiates the portion 10e from the other surface 10b, while the light sources 7a and 7b irradiate the portion 10e from one surface 10a.

A position of the portion 10e illustrated in FIG. 4A is a center portion of the paper 10, but the present invention is not limited to this, and may be an edge portion of the paper 10 or a portion between the center portion and the edge portion. It is considered that location dependence is low, and an example in which the position of the portion 10e is a predetermined position with respect to the paper 10 is considered.

More specifically, as long as the regions irradiated by the light sources 7a to 7c overlap each other, the light sources 7a to 7c may be disposed at any positions. In this sense, the portion 10e does not need to be a predetermined position on the paper 10, and for example, a configuration example is also considered in which the position varies for each paper 10.

The portion 10e is one location with respect to the paper 10, but an example in which a plurality of locations are provided is also considered.

Here, in FIG. 4A, fibers at one surface 10a of the paper 10 are schematically illustrated by thick lines.

In a case of manufacturing the paper 10, a paper stock containing fibers is dispersed in water and diluted in a paper machine, and then placed on, for example, a wire that moves in one direction, water is dropped, and the paper stock is vibrated to be leveled and formed into a uniform sheet.

By cutting the continuous paper, which is the paper manufactured in this manner, into a predetermined dimension, the paper 10 as a sheet-fed paper is obtained.

The fibers of the paper 10 are oriented in various directions with respect to one side 10c and the other side 10d, and are also oriented in various directions with respect to a thickness direction.

Meanwhile, in the paper manufacturing process described above, most of the elongated fibers have a feature of easily being bonded to each other to form a layer while being arranged in a matrix (arrangement) in a flow direction. A direction of paper that is the same as a traveling direction of a wire in the paper machine is referred to as "grain direction", "T direction", or "machine direction (MD)". In addition, a direction of paper that intersects the traveling direction of the wire is sometimes referred to as "cross grain direction" or "Y direction" or "CD" (cross direction).

The "grain direction" refers to a direction in which fibers flow parallel to a long side of the entire paper (for example, reference numeral 10c in FIGS. 4A and 4B). The "cross grain direction" refers to a direction in which fibers flow parallel to a short side of the entire paper (for example, reference numeral 10d in FIGS. 4A and 4B).

The light detector 7d captures an image of the portion 10e in a case of being irradiated with light from the light source 7a and an image of the portion 10e in a case of being irradiated with light from the light source 7b. In addition, the light detector 7d captures an image of the portion 10e in a case of irradiation with light from the light source 7c.

In the image obtained by the light source 7a, a fiber orientation in the grain direction is remarkably exhibited, and in the image obtained by the light source 7b, a fiber orientation in the cross grain direction is remarkably exhibited.

Therefore, the fiber orientation can be checked in a plurality of directions, such as the grain direction and the cross grain direction, by the image obtained by the light source 7a and the image obtained by the light source 7b. The fiber orientation described herein refers to an orientation state of the fibers in the paper 10. In addition, the orientation described herein refers to an orientation of the fiber.

In the images obtained by the light sources 7a and 7b, the fiber orientation can be checked by observing shadows of the fiber caused by the light emitted to the surface. As described above, the image acquisition portion 27a of the information processing portion 27 performs such a check.

In addition, in the image of the light source 7c, a fiber density in a thickness direction can be checked. In the image obtained by the light source 7c, the fiber density or air permeability can be checked by observing the transmission of light from the back surface. As described above, the image acquisition portion 27a of the information processing portion 27 performs such a check. The air permeability described herein refers to a rate at which air escapes from one surface of the paper 10 to the other surface.

In this manner, in a case where the paper 10 is irradiated with light in a direction orthogonal to the fiber orientation direction of the paper 10, a captured image having high contrast in which a state of the fibers of one surface 10a of the paper 10 is emphasized is obtained. By performing imaging in the grain direction and the cross grain direction, the fiber orientation for each of the grain direction and the cross grain direction can be checked.

Further, the fiber density in the thickness direction can be checked by irradiating the other surface 10b of the paper 10 with light.

Any one of the image obtained by the light source 7a or the image obtained by the light source 7b is an example of a first image in a case where the surface of the paper 10 is irradiated with light in one direction, and the other is an example of a second image in a case where the surface of the paper 10 is irradiated with light in another direction.

For example, in a case where the image by the light source 7a is an example of the first image, the cross grain direction (a direction in which the other side 10d extends) is an example of one direction, and the grain direction (a direction in which one side 10c extends) is an example of the other direction.

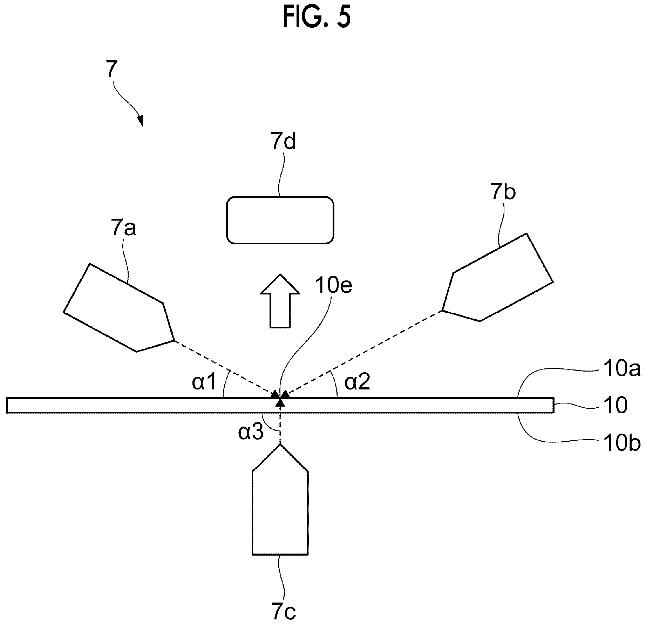

FIG. 5 is a front view schematically illustrating a relationship between the light sources 7a to 7c, the light detector 7d, and the paper 10, and corresponds to FIG. 4B described above.

As described above, the light sources 7a and 7b are located on one surface 10a side of the paper 10, and the light source 7c is located on the other surface 10b side of the paper 10.

As illustrated in FIG. 5, the light detector 7d is located on one surface 10a side of the paper 10.

Therefore, the light detector 7d captures an image of reflected light of the light sources 7a and 7b, and captures an image of transmitted light of the light source 7c.

As illustrated in FIG. 5, the light of the light source 7a is incident on one surface 10a of the paper 10 at an incidence angle α1. In addition, the light of the light source 7b is incident on one surface 10a of the paper 10 at an incidence angle α2.

Further, the light of the light source 7c is incident on the other surface 10b of the paper 10 at an incidence angle α3.

The incidence angles α1 and α2 described herein can be referred to as surface incidence angles, and the incidence angle α3 can be referred to as a back surface incidence angle.

It is considered that any value in a range of, for example, 10 to 20 degrees is adopted as the incidence angles α1 and α2. More specifically, it is considered that the incidence angle α1 and the incidence angle α2 have the same value.

For the incidence angle α3, for example, a value of 90 degrees is considered to be adopted.

The paper 10 is placed on a table (not illustrated). In addition, a pressing member (not illustrated) is placed on one surface 10a of the paper 10. That is, the paper 10 is sandwiched between a base (not illustrated) and the pressing member (not illustrated). Therefore, the paper 10 is stabilized. The paper 10 in this case is in a stationary state.

The base and the pressing member (not illustrated) are provided such that at least the portion 10e of the paper 10 is removed so as not to interfere with optical paths of the light sources 7a to 7c.

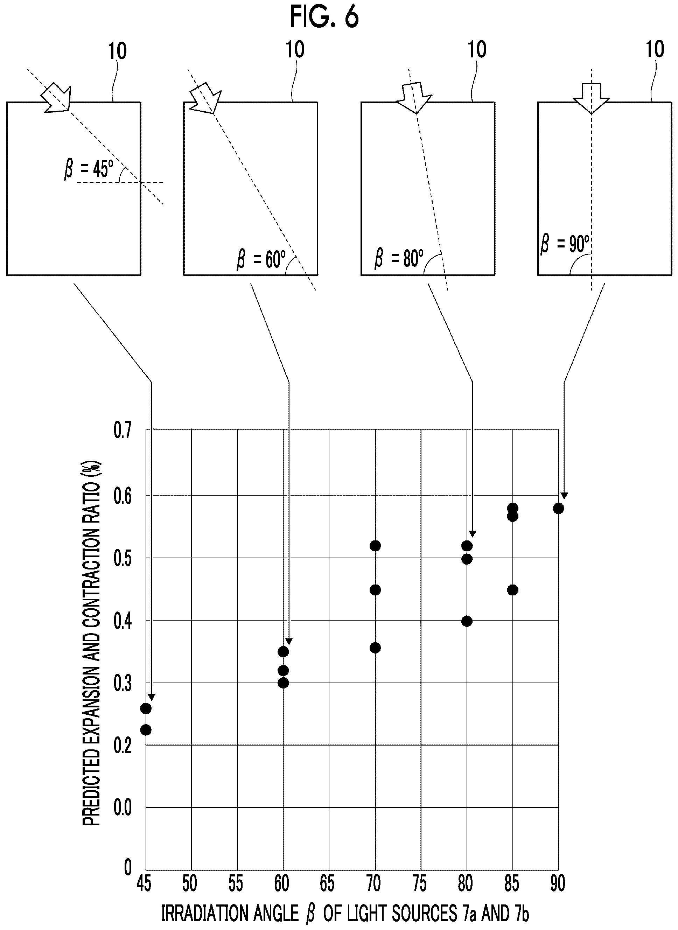

FIG. 6 is a graph describing a relationship between an irradiation angle β of the light sources 7a and 7b and a predicted expansion and contraction ratio, in which a vertical axis represents the predicted expansion and contraction ratio (%), and a horizontal axis represents the irradiation angle β (degree) of the light sources 7a and 7b. The irradiation angle β described herein refers to the irradiation angle β of the light sources 7a and 7b, which detect a fiber orientation, with respect to the paper side.

The irradiation angle β refers to an angle of light in a case of detecting the fiber orientation direction of the paper 10.

Since the light source 7a performs irradiation in the cross grain direction (a direction in which the other side 10d (see FIG. 4A) extends), the irradiation angle β is an angle with respect to one side 10c (see FIG. 4A). The irradiation angle β in such a case is an example of a first angle.

Since the light source 7b performs irradiation in the grain direction (a direction in which one side 10c (see FIG. 4A) extends), the irradiation angle β is an angle with respect to the other side 10d (see FIG. 4A). The irradiation angle β in such a case is an example of a second angle.

In the graph in FIG. 6, a case where the irradiation angle β is 45 degrees, a case where the irradiation angle β is 60 degrees, a case where the irradiation angle β is 70 degrees, a case where the irradiation angle β is 80 degrees, a case where the irradiation angle β is 85 degrees, and a case where the irradiation angle β is 90 degrees are illustrated.

As illustrated in FIG. 6, in a case where the irradiation angle β is 90 degrees, a predicted expansion and contraction ratio is the highest at 0.57. As the irradiation angle β deviates from 90 degrees, prediction accuracy is decreased. In a case of the predicted expansion and contraction ratio of 0.57, the expansion and contraction ratio is approximately 0.51 with an error of 10% and is approximately 0.46 with an error of 20%. In a case where the irradiation angle β is 70 degrees, two of the three predictive values are within 20%, in terms of the error of 20%.

From the above, it is required that the irradiation angle β is, for example, any value in a range from 70 degrees to 90 degrees. In other words, the irradiation angle β is any angle in a range from 70 degrees to 90 degrees. In addition, a case where the irradiation angle β of the light source 7a and the irradiation angle β of the light source 7b are set to the same value or different values is considered.

The media sensor 7 is configured such that the irradiation angle β is 90 degrees, but the influence on the predicted expansion and contraction ratio is within an allowable range as long as the irradiation angle β is within a range of 70 degrees as an error. In a case where any value in a range from 70 degrees to 90 degrees is set, it is not assumed that the value is changed thereafter.

In this manner, the value of the irradiation angle β can be set to any of the ranges determined based on the error with respect to the expansion and contraction ratio corresponding to the irradiation angle β. The error described herein can be an error ratio. A case where the irradiation angle β of any one of the light source 7a or the light source 7b is set to be the same as the irradiation angle β of the other is considered, and a case where the irradiation angles β are different from each other is also considered.

Since a relationship between the predicted expansion and contraction ratio and the irradiation angle β is caused by the fiber orientation and the fiber density of the paper 10, it can be said that the relationship is highly dependent on a type of paper.

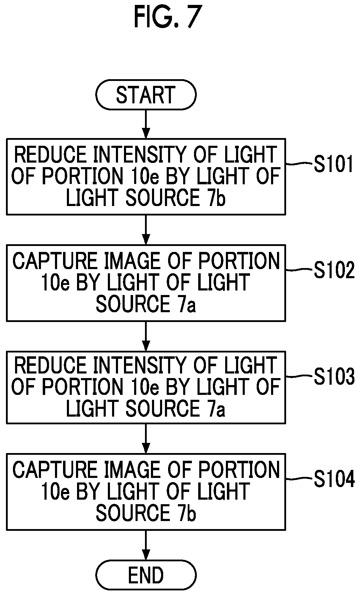

FIG. 7 is a flowchart illustrating an example of a processing procedure in a case where an image of the portion 10e is captured with light from the light sources 7a and 7b.

In the processing procedure example illustrated in FIG. 7, a case where an image of the portion 10e is captured by light of the light source 7a and then an image of the portion 10e is captured by light of the light source 7b is illustrated as an order of the imaging (see FIG. 5) of the portion 10e by the light detector 7d with the light of the light sources 7a and 7b.

The portion 10e is a part of one surface 10a of the paper 10, which is irradiated with the light from the light source 7a and the light from the light source 7b (see FIGS. 4A or FIG. 5), and is an example of an irradiation position. The portion 10e is irradiated with light from the light source 7c from the other surface 10b of the paper 10 (see in FIG. 4B or FIG. 5).

First, an intensity of light of the portion 10e by light of the light source 7b is set to be lower than an intensity of light of the portion 10e by light of the light source 7a (step S101). An image of the portion 10e is captured by the light of the light source 7a in a state in which an intensity of light of the light source 7b is reduced (step S102).

Then, the intensity of light of the portion 10e by the light of the light source 7a is set to be lower than the intensity of light of the portion 10e by the light of the light source 7b (step S 103). An image of the portion 10e is captured by the light of the light source 7b in a state in which the intensity of light of the light source 7a is reduced (step S104).

In this manner, as an irradiation condition at imaging of the light sources 7a and 7b, in a case where an imaging is performed by any one of the light sources 7a or 7b, the intensity of light of the other light source is reduced. In this manner, a difference in intensity of light at the portion 10e is used as the irradiation condition to prevent a shadow of the portion 10e from being canceled out by one light and the other light.

Here, in the light sources 7a and 7b, a supplied current (mA) is proportional to a luminous intensity (cd) of output light. In a case where the current is increased, the luminous intensity is increased, and in a case where the current is decreased, the luminous intensity is decreased. The luminous intensity (cd) described herein is a unit (candela) indicating an intensity of light.

The intensity of light of the portion 10e described above is a unit (lumen·second) indicating the total amount of luminous flux in a region of the portion 10e. The intensity of light is increased as the luminous intensity of the output light is increased, and the intensity of light is decreased as the luminous intensity of the output light is decreased.

As described above, in a case where the imaging is performed by one light source of the light sources 7a and 7b, the control of reducing the intensity of light of the other light source without turning off the other light source is adopted. The reason why the light source is not turned off is that fiber state evaluation accuracy is prevented from being lowered since the current fluctuates and the intensity of light is changed, and a shadow intensity is changed in a case where lighting is repeated.

In a case of performing imaging with one of the light sources 7a and 7b, a control of reducing the intensity of light without turning off the light source 7c may be adopted.

In this manner, in the processing procedure example illustrated in FIG. 7, for example, in a case where a fiber orientation of one side 10c (see FIG. 4A) is measured (imaged) with the light source 7a, the control of reducing the intensity of light of the other light source 7b is performed. More specifically, in this case, the other light source 7b is controlled not to be turned off.

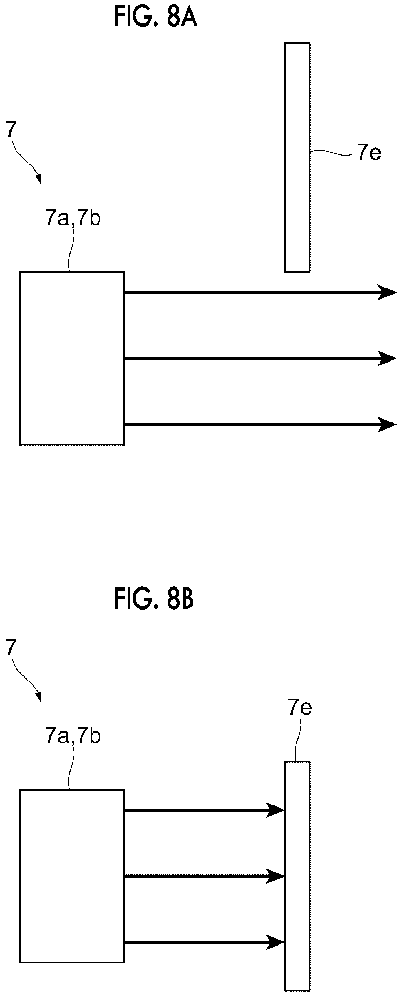

More specifically, as a method of reducing the intensity of light (see steps S101 and 103), a case where a current supplied to the other side is reduced to reduce a luminous intensity of the light is considered. In addition, it is also considered that a shutter member 7e (see FIGS. 8A and 8B) to be described below is disposed between the light source 7a and the portion 10e and between the light source 7b and the portion 10e.

FIGS. 8A and 8B are diagrams describing a configuration example of the shutter member 7e, in which FIG. 8A illustrates a case where light from the light sources 7a and 7b is not blocked by the shutter member 7e, and FIG. 8B illustrates a case where the light from the light sources 7a and 7b is blocked by the shutter member 7e.

The shutter member 7e illustrated in FIGS. 8A and 8B is a movable member that can suppress an intensity of light with which the portion 10e (see FIGS. 4A and 4B) is irradiated. The shutter member 7e is selectively movable to a position (see FIG. 8B) at which optical paths of the light sources 7a and 7b are blocked and a position (see FIG. 8A) at which the optical paths of the light sources 7a and 7b are not blocked.

By blocking the light with the shutter member 7e, the same effect as in a case where the luminous intensity of the light is reduced can be obtained. That is, the control of reducing the luminous intensity of the light is not performed. In addition, in the case of the shutter member 7e, it is not necessary to perform the control to reduce the supplied current, and a control load is reduced.

The shutter member 7e is an example of one member and an example of another member.

Next, a processing example in the information processing portion 27 (see FIG. 3) will be described.

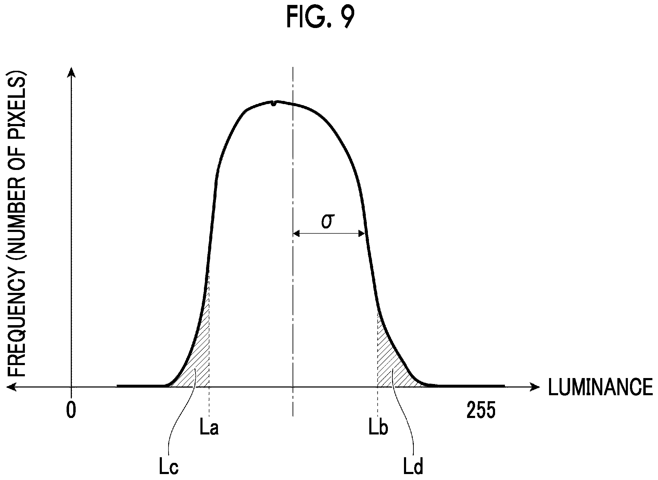

FIG. 9 is a histogram describing a process of the image acquisition portion 27a of the information processing portion 27, and a vertical axis is a frequency (the number of pixels) and a horizontal axis is a class of luminance (cd/m²).

As described above, the image acquisition portion 27a (see FIG. 3) acquires each image of the portion 10e (for example, see FIG. 5) of the paper 10 by the light sources 7a to 7c. Then, a fiber orientation indicating an orientation state of fibers in the paper 10 is checked from a plurality of directions, by using a plurality of acquired images.

In FIG. 9, luminances La and Lb (La < Lb) are illustrated as a range of a standard deviation ±σ with respect to an average. In addition, in FIG. 9, ranges Lc and Ld outside the range of the standard deviation ±σ are indicated by diagonal lines. The average is a standard luminance.

Most of pixels have luminances in the range of the standard deviation ±σ (that is, a range of the luminance La or more and the luminance Lb or less), and there are also pixels in the ranges Lc and Ld of the diagonal lines depending on a fiber state of the paper 10.

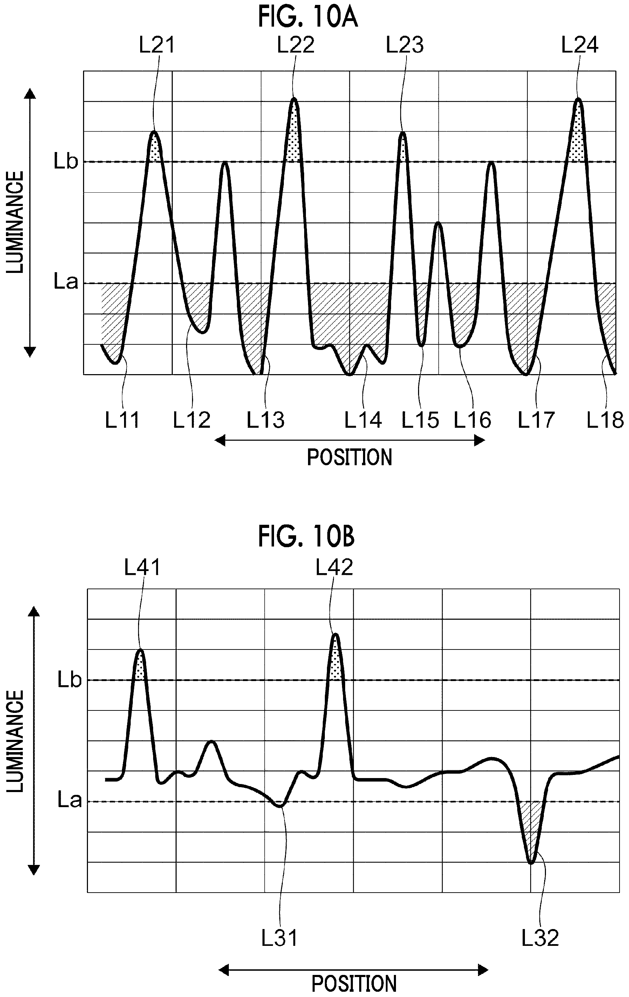

FIGS. 10A and 10B are graphs illustrating a fiber orientation of an image by light reflected by the paper 10, in which FIG. 10A illustrates an image by the light source 7a (for example, see FIGS. 4A and 4B) and FIG. 10B illustrates an image by the light source 7b (see FIGS. 4A and 4B). In both FIGS. 10A and 10B, a vertical axis is a luminance, and a horizontal axis is a position of the paper 10. More specifically, the position of the paper 10 in FIG. 10A is a position related to one side 10c in the grain direction (see FIGS. 4A and 4B), and the position of the paper 10 in FIG. 10B is a position related to the other side 10d in the cross grain direction (see FIGS. 4A and 4B).

In the graph of the fiber orientation in the grain direction illustrated in FIG. 10A, a pattern of diagonal lines or hatching is added to a portion deviating from a range (range of standard deviation ±σ) between the luminance La and the luminance Lb. That is, portions L11, L12, L13, L14, L15, L16, L17, and L18 in FIG. 10A have a luminance lower than the luminance La. In addition, portions L21, L22, L23, and L24 have a luminance higher than the luminance Lb.

In addition, in the graph of the fiber orientation in the cross grain direction illustrated in FIG. 10B, a portion deviating from a range (range of standard deviation ±σ) between the luminance La and the luminance Lb is marked with a pattern. That is, portions L31 and L32 in FIG. 10B have a luminance lower than the luminance La. In addition, portions L41 and L42 have a luminance higher than the luminance Lb.

In this manner, the portions L11 to L18 and L31 to L32 having a luminance lower than the luminance La and the portions L21 to L24 and L41 to L42 having a luminance higher than the luminance Lb are out of the range of the standard deviation ±σ.

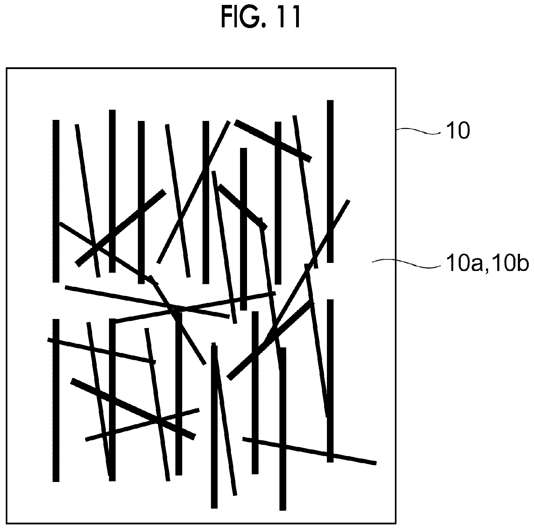

FIG. 11 is a view illustrating a fiber density of the paper 10 in a thickness direction, in which fibers are schematically illustrated by thick lines. The thickness direction described herein is an example of a direction different from a plurality of directions, and can also be referred to as a through-thickness direction.

The view illustrated in FIG. 11 is an image as a detection result of the light detector 7d with light of the light source 7c (for example, FIG. 5), and is a plan view as viewed from one surface 10a of the paper 10 or a bottom view as viewed from the other surface 10b. Therefore, overlapping of the fibers in the thickness direction in the portion 10e (for example, see FIG. 5) of the paper 10 can be checked.

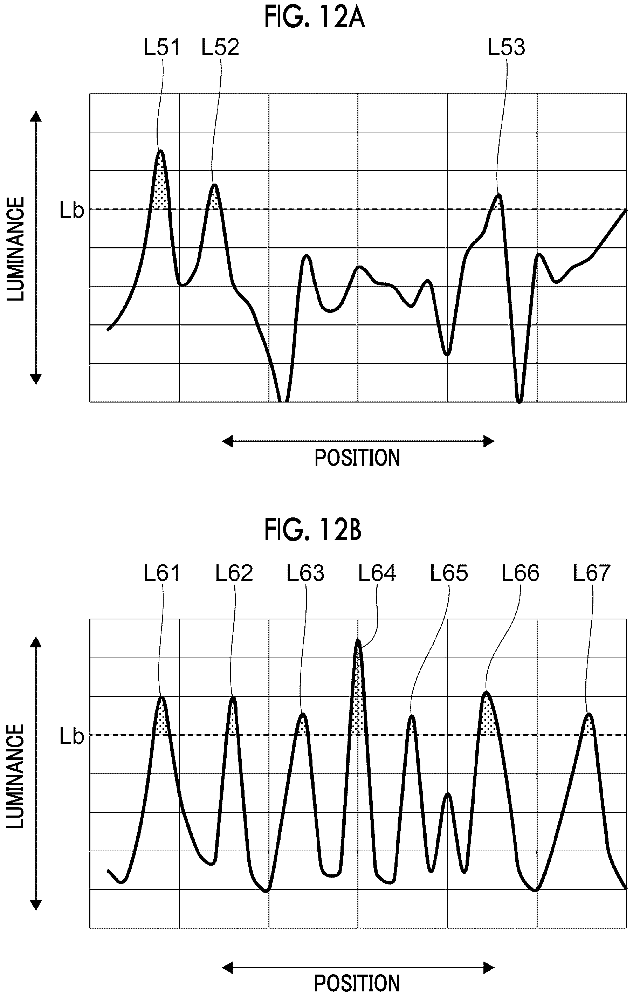

FIGS. 12A and 12B are graphs illustrating a fiber density of an image by light transmitted through the paper 10, and FIGS. 12A and 12B illustrate a case of an image by the light source 7c (for example, see FIGS. 4A and 4B). FIG. 12A illustrates a case of the paper 10 having a high fiber density, and FIG. 12B illustrates a case of the paper 10 having a low fiber density. The light transmitted through the paper 10 is light from the light source 7c.

In the graph in FIG. 12A in a case where the fiber density is high, a portion higher than the luminance Lb (see FIG. 9) is marked with a pattern. That is, portions L51, L52, and L53 in FIG. 12A have a luminance higher than the luminance Lb.

In addition, in the graph illustrated in FIG. 12B in a case where the fiber density is low, a portion higher than the luminance Lb (see FIG. 9) is hatched. That is, portions L61, L62, L63, L64, L65, L66, and L67 in FIG. 12B have a luminance higher than the luminance Lb.

As described above, a shadow of the fiber due to the light of the light sources 7a and 7b (see FIG. 5) with which a surface of the paper 10 is irradiated is observed. In addition, a shadow of the fiber caused by the light transmitted through the paper 10 is observed.

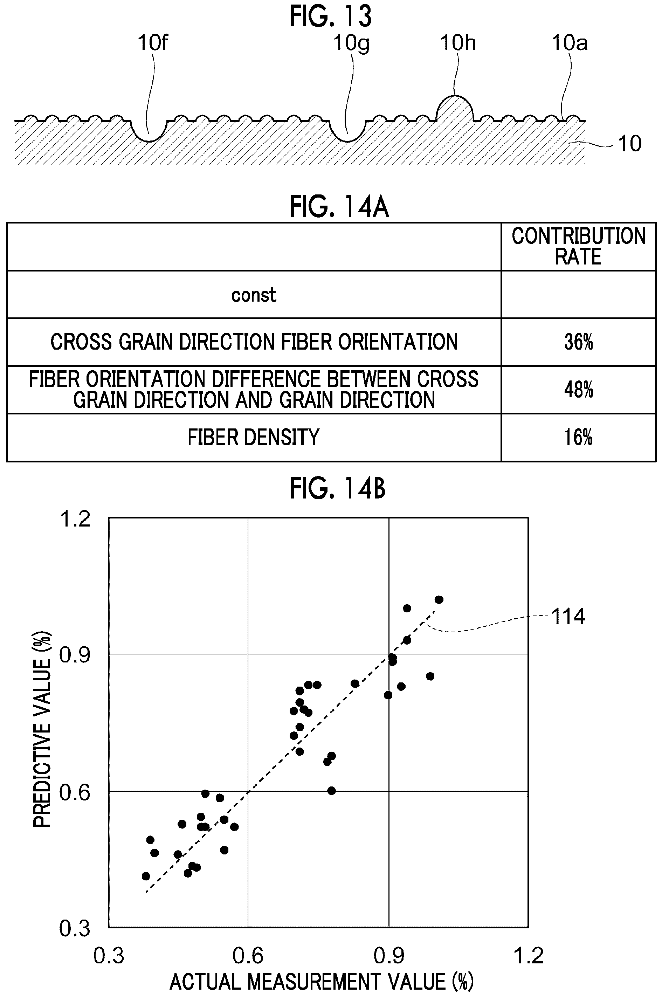

FIG. 13 is a cross-sectional view illustrating a state of roughness (surface roughness) on one surface 10a of the paper 10.

One surface 10a of the paper 10 illustrated in FIG. 13 has fine roughness, and has a larger protruding portion 10h or has larger recess portions 10f and 10g.

The light from the light sources 7a and 7b (see FIG. 5), which is reflected by one surface 10a of the paper 10, has a high luminance in the protruding portion 10h, while the light has a low luminance in the recess portions 10f and 10g.

The portions L21 to L24 and L41 to L42 (see FIGS. 10A and 10B) described above correspond to the protruding portion 10h in this case. In addition, the portions L11 to L18 and L31 to L32 (see FIGS. 10A and 10B) correspond to the recess portions 10f and 10g.

In addition, the light emitted from one surface 10a and transmitted through the paper 10, which is the light from the light source 7c (see FIG. 5), is high in the recess portions 10f and 10g. The portions L51 to L53 and L61 to L67 (see FIGS. 12A and 12B) correspond to the recess portions 10f and 10g in this case.

Here, the number of images of ranges Lc and Ld (see FIG. 9) that are excluded as being out of the range of the standard deviation ±σ (see FIG. 9) is focused. In a case where a ratio of the number of excluded pixels to the total number of images is defined as an exclusion region ratio, a fiber orientation is checked by the exclusion region ratio in the light sources 7a to 7c. That is, it is possible to specify which of the grain direction and the cross grain direction is a flow direction during paper making. The exclusion region ratio indicates a tendency of large roughness.

The orientation state of the fibers in the grain direction and the cross grain direction can be checked by the light sources 7a and 7b, and an overlapping state of the fibers in an oblique direction can be checked by the light source 7c. An image obtained by the light sources 7a to 7c can be used to check the three-dimensional fiber state in three directions.

For example, in the case of the light of the light sources 7a and 7b, a large shadow is not generated in the cross grain direction (see FIG. 10B), but a large shadow is generated in the grain direction (see FIG. 10A). Since the roughness in the grain direction is more exhibited than in the cross grain direction, the flow direction during paper making of the paper 10 is the cross grain direction.

Next, a method of deriving an expansion and contraction ratio of the paper 10 from a fiber orientation will be described. The information processing portion 27 (see FIG. 3) executes a prediction model construction program. Such a program extracts a factor that contributes to the expansion and contraction ratio by a variable reduction method based on, for example, measurement data or an actual measurement value.

The variable reduction method described herein is one of methods of selecting a combination of variables with high explanatory power in multiple regression analysis, and sequentially screening out variables from a prepared group of variables to increase the accuracy of the multiple regression analysis. The regression analysis is a method of finding a coefficient of a relational expression such that an actual value and a theoretical value are close to each other.

More specifically, the regression analysis is executed by using all independent variables, the results are checked, and the independent variables are checked. The analysis is ended in a case where there is no checked independent variable. In a case where there is an independent variable that is checked, a table excluding the independent variable is created, and the regression analysis is executed again with the newly created table.

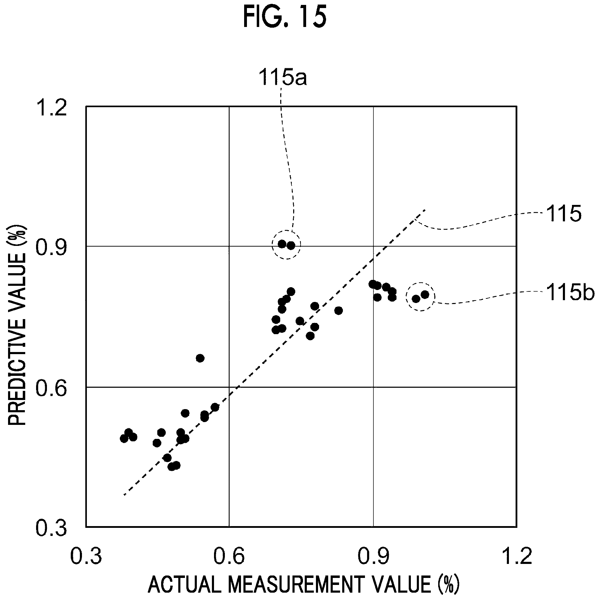

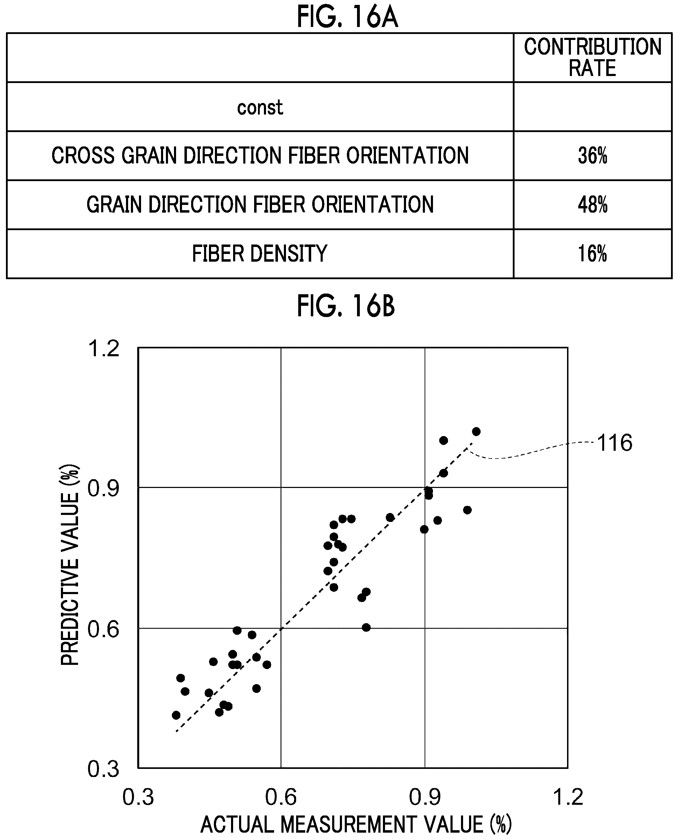

FIGS. 14A and 14B are diagrams describing prediction accuracy in a case of predicting an expansion and contraction ratio in a cross grain direction, in which FIG. 14A is a table illustrating an extraction result and FIG. 14B is a matrix diagram illustrating a correlation between a predictive value of the expansion and contraction ratio and an actual measurement value of the expansion and contraction ratio, respectively. FIG. 15 is a diagram describing prediction accuracy in a case of predicting the expansion and contraction ratio in the cross grain direction. FIGS. 16A and 16B are diagrams describing prediction accuracy in a case of predicting the expansion and contraction ratio in the cross grain direction, in which FIG. 16A is a table illustrating an extraction result and FIG. 16B is a matrix diagram illustrating a correlation between a predictive value of the expansion and contraction ratio and an actual measurement value of the expansion and contraction ratio, respectively.

The graph illustrated in FIGS. 14A and 14B illustrates a correlation between the actual measurement value and the predictive value of the expansion and contraction ratio, in an example focusing on a fiber orientation, a fiber orientation difference between the cross grain direction and the grain direction, and a fiber density by a light source from the three directions illustrated in FIG. 5. A horizontal axis is an actual measurement value of the expansion and contraction ratio, and is obtained by an expansion and contraction ratio measuring device in the related art. A vertical axis is a predictive value of the expansion and contraction ratio. Points of the predictive value obtained by performing the multiple regression analysis from the fiber state described above are close to the actual measurement value. It can be seen that the prediction close to the actual measurement value can be made by using these.

The table illustrated in FIG. 14A illustrates a case where three factors of a cross grain direction fiber orientation, a fiber orientation difference between the cross grain direction and the grain direction, and a fiber density are extracted. The fiber orientation difference between the cross grain direction and the grain direction described herein refers to a difference between a fiber orientation in the cross grain direction and a fiber orientation in the grain direction.

A contribution rate of the cross grain direction fiber orientation is relatively high at 36%, but a contribution rate of the fiber orientation difference between the cross grain direction and the grain direction is 48%, which is a value close to 50%, so that it can be understood as a major factor.

In a case where x is an actual measurement value and y is an expected value, y = 0.9938x is obtained as a straight line 114 from points plotted in FIG. 14B. All the plotted points is close to the straight line 114. Therefore, it can be seen that, in a case where the straight line 114 is used as the prediction model, it is possible to perform prediction fairly close to the actual measurement value.

On the other hand, in a case where x is an actual measurement value and y is an expected value, y = 0.977x is obtained as a straight line 115 from points plotted in FIG. 15. In the plotted points, an error is large in regions 115a and 115b having a high expansion and contraction ratio.

The table illustrated in FIG. 16A illustrates a case where three factors of a cross grain direction fiber orientation, a grain direction fiber orientation, and a fiber density are extracted. That is, in FIG. 16A, the fiber orientation difference between the cross grain direction and the grain direction in FIG. 14A is replaced with the grain direction fiber orientation.

As illustrated in FIG. 16A, a contribution rate of the grain direction fiber orientation is 48%, as in the contribution rate of the fiber orientation difference between the cross grain direction and the grain direction (see FIG. 14A).

From points plotted in FIG. 16B, y = 0.9938x is obtained as a straight line 116. All the plotted points is close to the straight line 116. Therefore, it can be seen that, in a case where the straight line 116 is used as the prediction model, it is possible to perform prediction fairly close to the actual measurement value.

In this manner, by extracting any two or more factors of the cross grain direction fiber orientation, the grain direction fiber orientation, and the fiber orientation difference between the cross grain direction and the grain direction, it is possible to obtain a prediction model capable of predicting a value close to an actual measurement value.

The cross grain direction fiber orientation is an example of a first fiber orientation checked by a first image, the grain direction fiber orientation is an example of a second fiber orientation checked by a second image, and the fiber orientation difference between the cross grain direction and the grain direction is an example of a difference between the first fiber orientation and the second fiber orientation. The expansion and contraction ratio is derived by a combination of any two or more of these three fiber orientations.

In addition, the expansion and contraction ratio may be derived by a combination including the fiber density and at least one of the cross grain direction fiber orientation, the grain direction fiber orientation, or the difference between the cross grain direction fiber orientation and the grain direction fiber orientation.

It is conceivable to adopt such a combination of the predictive variables.

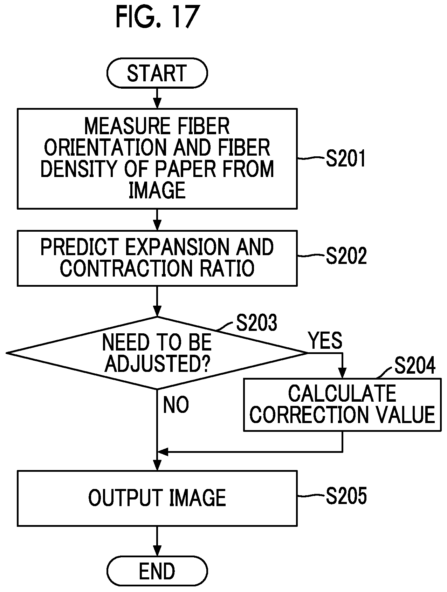

FIG. 17 is a flowchart illustrating a processing example of correcting a size of an image to be formed on the paper 10 (for example, see FIG. 1) according to an expansion and contraction ratio of the paper 10. In the description in FIG. 17, the "paper 10" is simply referred to as "paper".

In the processing example illustrated in FIG. 17, the image acquisition portion 27a (see FIG. 3) of the information processing portion 27 measures a fiber orientation and a fiber density of the paper from the acquired image (step S201). Then, the expansion and contraction ratio deriving portion 27b (see FIG. 3) predicts the expansion and contraction ratio of the paper by using a prediction model (step S202).

It is determined whether or not the image to be formed on the paper needs to be adjusted based on the predicted expansion and contraction ratio (step S203). It is considered that such determination is performed by comparison with a predetermined threshold value. That is, in a case where the predicted expansion and contraction ratio is higher than the threshold value or lower than the threshold value, it is determined that the adjustment of the image is necessary, and in other cases, it is determined that the adjustment of the image is not necessary.

In a case where it is determined that the adjustment of the image is necessary (Yes in step S203), the image correction portion 27c (see FIG. 3) calculates a correction value (step S204). The correction value to be calculated will be described below.

The image processing portion 22 (refer to FIG. 1) adjusts the image size by using the calculated correction value, and outputs the adjusted image (step S205). That is, in a case where the expansion and contraction ratio exceeds 100%, adjustment of enlarging the image is performed, and in a case where the expansion and contraction ratio is lower than 100%, adjustment of reducing the image is performed.

In a case where it is determined that the adjustment of the image is not necessary (No in step S203), the image processing portion 22 (refer to FIG. 1) outputs the image without performing the size correction (step S205).

In this manner, in a case where the size of the image is adjusted according to the present exemplary embodiment, for example, since adjustment chart printing is not necessary, the measurement can be performed for each printing target paper. That is, the exemplary embodiment can also cope with a variation between the papers. In addition, in the related art, it takes time to evaluate the expansion and contraction ratio of the paper 10, but in the case of the present exemplary embodiment, it is possible to evaluate the expansion and contraction ratio in a shorter time than in the related art. Further, with the present exemplary embodiment, it is possible to respond even without experience necessary for adjustment. The expansion and contraction ratio of the paper 10 is easily specified on the site.

Here, various specific examples of the calculation of the correction value (step S204 in FIG. 17) will be described.

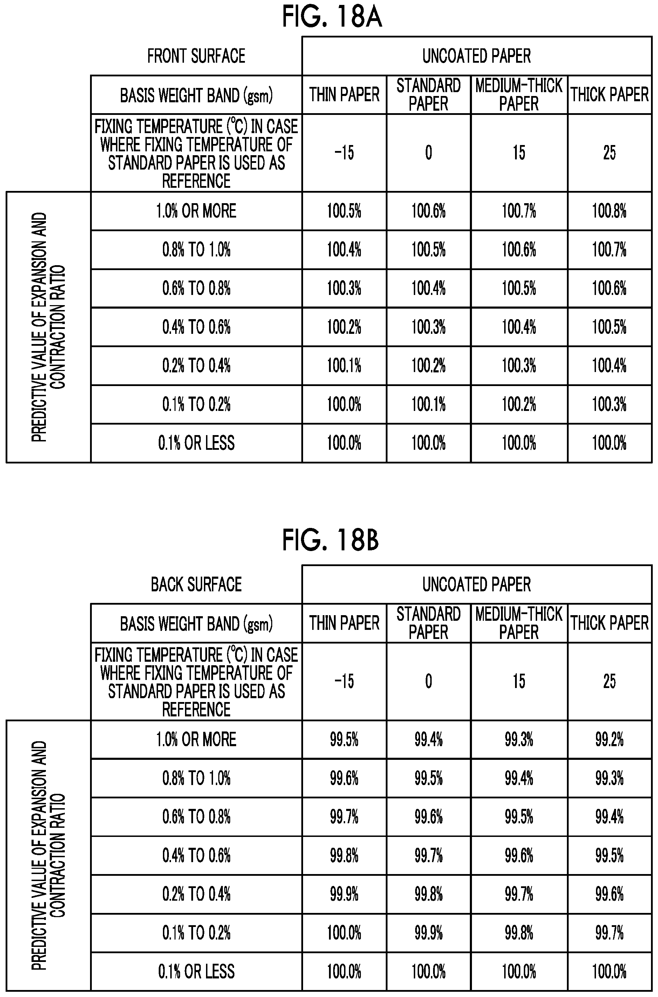

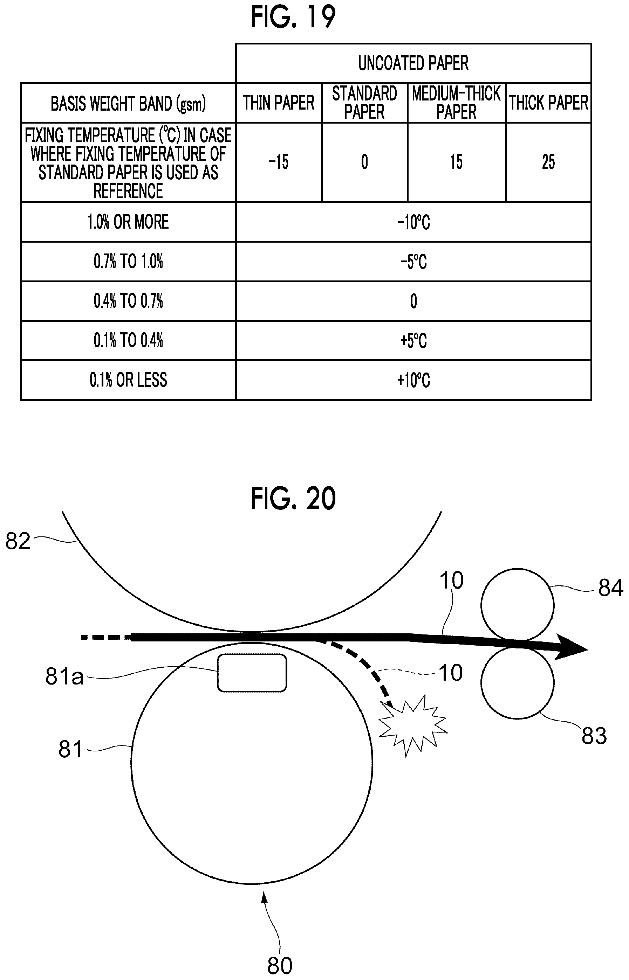

First, as a first specific example, a case where the deviation amount of an image is predicted from a predictive value of an expansion and contraction ratio and an image size is corrected before printing will be described with reference to FIGS. 18A and 18B. In addition, as a second specific example, a case where the deformation amount of the paper 10 is predicted from a predictive value of an expansion and contraction ratio and a fixing temperature is adjusted will be described with reference to FIG. 19. The fixing temperature described herein refers to a temperature at which the image formed on the paper 10 is fixed, and is controlled based on the expansion and contraction ratio.

FIGS. 18A and 18B are tables describing a first specific example of the calculation of the correction value (step S204 in FIG. 17), in which 18A is a case of printing on a front surface and 18B is a case of printing on a back surface. That is, in a case of performing single-sided printing, a correction value in FIG. 18A is used, and in a case of performing two-sided printing, correction values in FIGS. 18A and 18B are used.

The paper 10 is a type of paper called uncoated paper. Data of the correction values illustrated in FIGS. 18A and 18B is stored in, for example, the ROM 21d (see FIG. 2).

In both FIGS. 18A and 18B, each item of a basis weight band, a fixing temperature, and a predictive value of an expansion and contraction ratio is arranged from the top to the bottom of the table.

The basis weight band (gsm) is divided into thin paper, standard paper, medium-thick paper, and thick paper. The "basis weight band" described herein refers to a section of a basis weight of the paper 10 and is represented by a set of a lower limit and an upper limit of the basis weight of each section. Meanwhile, in order to avoid complication of display, the "basis weight band" is represented by a name representing a thickness of the paper 10, such as "thin paper", "standard paper", "medium-thick paper", and "thick paper".

The fixing temperature is a temperature of the fixing device 80 (refer to FIG. 1) that fixes a printed image on the paper 10, and this temperature is automatically determined from a type of the paper 10. Meanwhile, in order to avoid the complication of display, the fixing temperature is represented by a positive or negative temperature difference in a case where a fixing temperature in a case of standard paper is used as a reference. The fixing temperature is set and changed depending on the basis weight, the paper type, a paper size, and an environment. In FIGS. 18A and 18B, a set value for each basis weight of the uncoated paper is presented.

The predictive values of the expansion and contraction ratio are classified into seven categories in an order of 1.0% or more, 0.8% to 1.0%, 0.6% to 0.8%, 0.4% to 0.6%, 0.2% to 0.4%, 0.1% to 0.2%, and 0.1% or less. Then, a scaling factor of the image is determined by a combination of each divided predictive value and the basis weight band.

The scaling factor described herein indicates a ratio of enlargement or reduction to a size of an original image. The scaling factor of 100% represents that the original image is not enlarged or reduced. In addition, the scaling factor having a value higher than 100% represents enlargement of the original image, and a value lower than 100% represents reduction of the original image.

For example, in FIG. 18A, the surface expansion and contraction ratio of 100.5% applied in a case where the predictive value of the scaling factor is 1.0% or more and the basis weight band is thin paper represents that the original image is enlarged to 100.5% (that is, increased by 0.5% from the original size).

A scaling factor of each cell in scaling factor information for the back surface illustrated in FIG. 18B is a value lower than the scaling factor of the corresponding cell in the scaling factor information for the front surface illustrated in FIG. 18A. This reflects that moisture of the paper 10 evaporates by heating in the fixing device 80 (see FIG. 1) during printing on the surface, and the paper 10 is contracted. As the paper 10 is thicker, the paper is more likely to contract. Therefore, a reduction in the scaling factor is large as compared to the front surface. The use of the scaling factor information illustrated in FIG. 18B makes it possible to make the size of the image printed on the back surface of the paper 10 equal to the size of the image printed on the front surface of the paper 10. Therefore, it is possible to match the sizes in the registration of the front and back sides.

Next, a second specific example will be described.

FIG. 19 is a table describing a second specific example of the calculation of the correction value (step S204 in FIG. 17). The paper 10 is a type of paper called uncoated paper. Data of the correction values illustrated in FIG. 19 is stored in, for example, the ROM 21d (see FIG. 2).

In FIG. 19, each item of a basis weight band, a fixing temperature, and a predictive value of an expansion and contraction ratio is arranged from the top to the bottom of the table.

The basis weight band (gsm) and the fixing temperature are the same as in the case in FIGS. 18A and 18B described above, and the description thereof will be omitted.

The predictive values of the expansion and contraction ratio are classified into five categories in an order of 1.0% or more, 0.7% to 1.0%, 0.4% to 0.7%, 0.1% to 0.4%, and 0.1% or less. Then, an adjustment temperature corresponding to each divided predictive value is determined.

The adjustment temperature is constant, regardless of the basis weight band. For example, in a case where the predictive value of the expansion and contraction ratio is 1.0% or more, the adjustment temperature is -10°C.

Here, curl of the paper 10 in a transport path will be described.

FIG. 20 is a diagram illustrating a curl state of the paper 10 in the fixing device 80.

The fixing device 80 illustrated in FIG. 20 includes a heating roll 81 and a pressurizing roll 82, which are roll-shaped members. A heating unit 81a is provided inside the heating roll 81. The pressurizing roll 82 is pressed by the heating roll 81. The heating roll 81 and the pressurizing roll 82 can be rotated in opposite directions to each other.

The paper 10 is transported between the heating roll 81 and the pressurizing roll 82. In this case, a toner resin of an image stored on the paper 10 is heated and melted, and the image is fixed on the paper 10.

In addition, transport rolls 83 and 84 for transporting the paper 10 are provided on a downstream side of the heating roll 81 and the pressurizing roll 82.

The paper 10 is transported along a paper transport path including a space between the heating roll 81 and the pressurizing roll 82 and a space between the transport roll 83 and the transport roll 84, as illustrated by a solid line in FIG. 20.

Meanwhile, in a case where the paper 10 is transported between the heating roll 81 and the pressurizing roll 82, deformation due to heating may occur. That is, the paper 10 is curled as indicated by a broken line in FIG. 20. The curl of the paper 10 can be said to be deformation in a case where one surface of the paper 10 has a larger area than the other surface.

That is, in a case where moisture evaporates during the heating in the fixing and the paper 10 is contracted, a paper surface temperature on the heating roll 81 side is high, and a difference in contraction (temperature difference) between the front and back surfaces occurs, so that the paper 10 curls. The higher the expansion and contraction ratio, the larger the curl.

The curl of the paper 10 causes a paper jam due to a deviation from the transport path during transport in the image forming apparatus 1 (see FIG. 1). This is a so-called peeling type jam. The peeling type jam may occur in a case where the paper 10 has no stiffness, or may occur due to the expansion and contraction caused by heating described above.

The expansion and contraction of the paper 10 due to the heating in the fixing device 80 will be described.

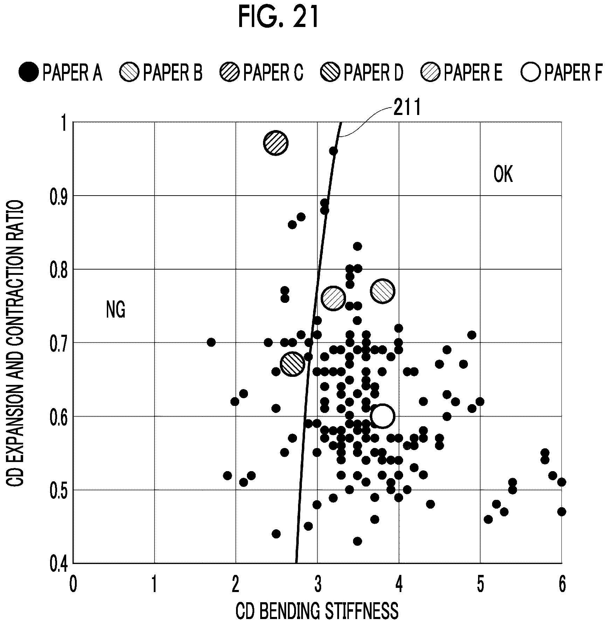

Each in FIGS. 21, 22, and 23 is a graph describing an occurrence state of a peeling type jam in a case where the paper 10 is heated, and each vertical axis is a CD expansion and contraction ratio and each horizontal axis is a CD bending stiffness.

The CD expansion and contraction ratio and the CD bending stiffness described herein indicate characteristics of the paper 10 in a direction intersecting a traveling direction of a paper machine. The bending stiffness is a physical quantity indicating stiffness of the paper 10 and can be referred to as bending rigidity.

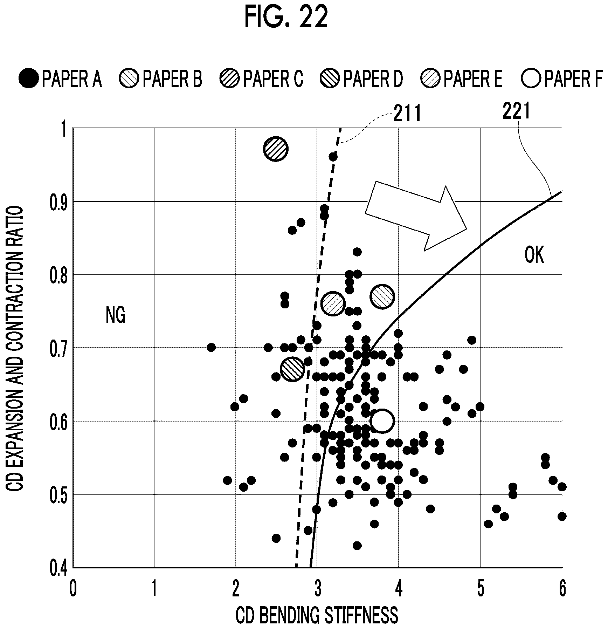

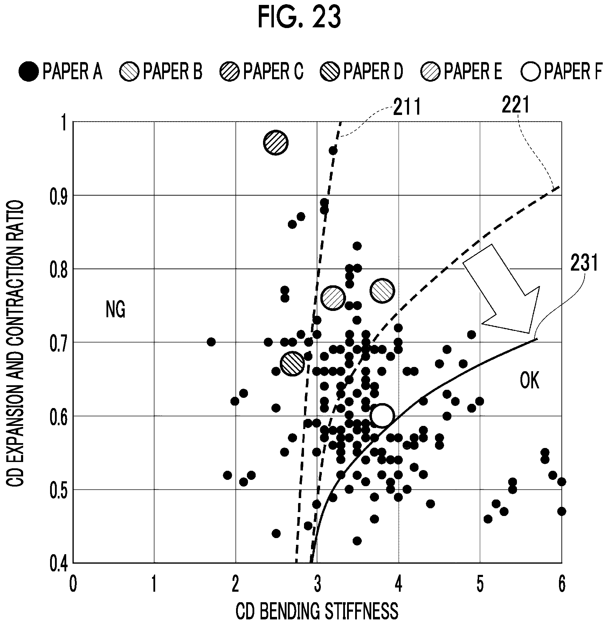

FIG. 21 illustrates a case of a fixing temperature t1, FIG. 22 illustrates a case of a fixing temperature t2 (t2 > t1), and FIG. 23 illustrates a case of a fixing temperature t3 (t3 > t2), respectively. A plurality of points plotted in each drawing are illustrated in a distinguishable manner for each type of paper 10. That is, paper A is represented by a black circle, paper B is represented by a thin diagonal line in a lower right corner, paper C is represented by a thick diagonal line in an upper right corner, paper D is represented by a thick diagonal line in the lower right corner, paper E is represented by a thin diagonal line in the upper right corner, and the paper F is represented by a white circle. The plurality of points in FIG. 21 are the same as the plurality of points in FIGS. 22 and 23.

In the graph illustrated in FIG. 21, a peeling type jam occurs in a left region of a line 211 indicated by a solid line (see "NG" display), and the peeling type jam does not occur in a right region (see "OK" display). Therefore, the paper 10 corresponding to the point plotted on the left side of the line 211 has the peeling type jam, while the paper 10 corresponding to the point plotted on the right side of the line 211 does not have the peeling type jam. Since FIG. 21 illustrates a case where a fixing temperature is relatively low, the line 211 tends to be caused by a value of the CD bending stiffness.

In FIG. 21, a coverage rate, which is a rate of the points on the right side of the line 211 to all the plotted points, is 85%.

In the graph illustrated in FIG. 22, a peeling type jam occurs in a left region of a line 221 indicated by a solid line (see "NG" display), and the peeling type jam does not occur in a right region (see "OK" display). In FIG. 22, the line 211 illustrated by the solid line in FIG. 21 described above is illustrated by the line 211 as a broken line.

In FIG. 22, since the fixing temperature t2 is higher than the fixing temperature in the case in FIG. 21, a proportion of occurrence of a peeling type jam is increased as the expansion and contraction ratio is high. A coverage rate in FIG. 22 is 60%, which is a lower value than that in the case in FIG. 21.

In the graph illustrated in FIG. 23, a peeling type jam occurs in a left region of a line 231 indicated by a solid line (see "NG" display), and the peeling type jam does not occur in a right region (see "OK" display). In FIG. 23, the line 211 illustrated by the solid line in FIG. 21 is represented by the line 211 as a broken line, and the line 221 illustrated by the solid line in FIG. 22 is represented by the line 221 as a broken line.

In FIG. 23, since the fixing temperature t3 is higher than the fixing temperature in the case in FIG. 22, a peeling type jam occurs in a case where the expansion and contraction ratio exceeds 0.7. A coverage rate in FIG. 23 is 25%, which is a lower value than that in the case in FIG. 22.

In order to prevent the peeling type jam caused by curling, it is necessary to check a position of the paper 10 to be used on the graphs in FIGS. 21 to 23 and to take measures according to the expansion and contraction ratio in the transport direction. That is, the control is performed such that the fixing temperature is changed according to the expansion and contraction ratio. In a case where the expansion and contraction ratio of the paper 10 is high, the fixing temperature is lowered.

In a case where the paper 10 has a low expansion and contraction ratio, an occurrence probability of the peeling type jam is low. Therefore, it is also considered to control the fixing temperature to be increased for the effect of improving a fixing failure.

In this manner, by grasping the expansion and contraction ratio of the paper 10 in advance and controlling the fixing temperature corresponding to the expansion and contraction ratio, it is possible to suppress the occurrence of the peeling type jam. Specifically, in a case where the expansion and contraction ratio is equal to or higher than a predetermined value, the fixing temperature is set to be lower than a predetermined temperature. As a result, curling is prevented.

In addition, in a case where the expansion and contraction ratio is equal to or lower than a predetermined value, the fixing temperature is set to be higher than a predetermined temperature. Therefore, fixing properties are improved.

Here, prediction accuracy in a case where the paper 10 is recycled paper will be described. That is, a paper type of the paper 10 is recycled paper.

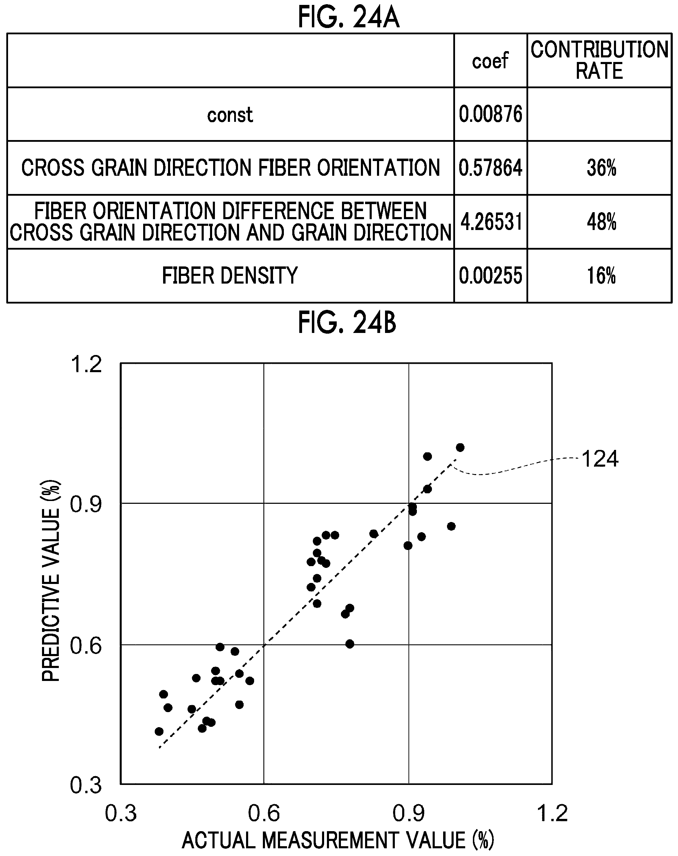

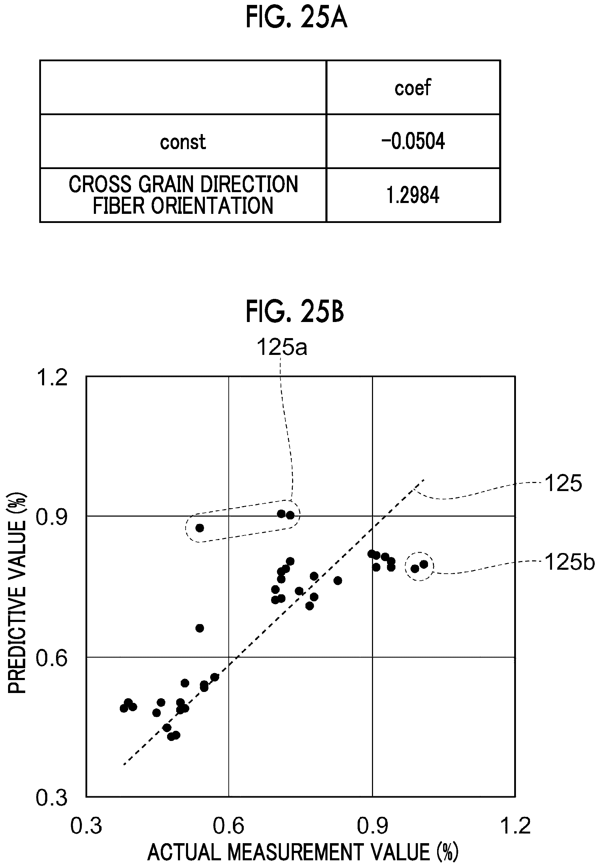

FIGS. 24A and 24B and FIGS. 25A and 25B are diagrams describing the prediction accuracy in a case of predicting the expansion and contraction ratio in the cross grain direction of recycled paper, in which FIG. 24A and FIG. 25A is a table illustrating an extraction result and FIG. 24B and FIG. 25B is a matrix diagram illustrating a correlation between a predictive value of the expansion and contraction ratio and an actual measurement value of the expansion and contraction ratio, respectively. FIGS. 24A and 24B and FIGS. 25A and 25B correspond to FIGS. 14A and 14B and 15 described above, and common description may be omitted.

The table illustrated in FIG. 24A illustrates a case where three factors of a cross grain direction fiber orientation, a fiber orientation difference between the cross grain direction and the grain direction, and a fiber density are extracted. The table illustrated in FIG. 25A illustrates a case where the cross grain direction fiber orientation is extracted.

As illustrated in FIG. 24B, it can be seen that, in a case where a straight line 124 is used as a prediction model, the prediction can be made to be quite close to an actual measurement value. On the other hand, in a case illustrated in FIG. 25B, even in a case where a straight line 125 is used as a prediction model, an error is large in regions 125a and 125b having a high expansion and contraction ratio.

In a case of measuring an expansion and contraction ratio of recycled paper, an error is large in a device of the related art.

The recycled paper has physical properties such as that a length of fibers is shorter than a length of fibers of the paper 10 other than the recycled paper and that the recycled paper contains a mixture of dust, dirt, and the like other than the fibers. In addition, as a cause of the large error, there is a probability that an orientation of the fibers cannot be correctly read due to the short fiber length, and there is a probability that the dust is erroneously determined as the fiber.

As a measure against such a cause, it is considered that, by adopting a configuration in which the fiber orientation is checked not only in one direction of the paper 10 but also in two directions as in the present exemplary embodiment, it is possible to prevent the fiber orientation from being erroneously recognized.

Although the exemplary embodiments of the present invention are described above, a technical scope of the exemplary embodiments of the present invention is not limited to the scope described in the exemplary embodiments described above. Various modifications or improvements are added to the exemplary embodiments described above within the technical scope of the exemplary embodiments of the present invention, and are apparent from the description of the claims.

The present invention can also be applied to a program and a program product.

### <Supplementary Notes>

(((1))) An information processing system comprising:
   a processor configured to:
   check a fiber orientation indicating an orientation state of fibers in paper from a plurality of directions; and
   derive an expansion and contraction ratio of the paper based on the checked fiber orientation.
(((2))) The information processing system according to (((1))),
   wherein the checking of the fiber orientation is performed by using a first image in a case where a surface of the paper is irradiated with light from one direction as the plurality of directions and a second image in a case where the surface of the paper is irradiated with light from another direction as the plurality of directions.
(((3))) The information processing system according to (((2))),
   wherein the irradiation with the light from the one direction is performed at a first angle with respect to the other direction, and
   the irradiation with the light from the other direction is performed at a second angle with respect to the one direction.
(((4))) The information processing system according to (((3))),
   wherein the first angle is any angle in a range determined based on an error with respect to the expansion and contraction ratio corresponding to an angle with respect to the other direction, and
   the second angle is any angle in a range determined based on an error with respect to the expansion and contraction ratio corresponding to an angle with respect to the one direction.
(((5))) The information processing system according to (((3))),
   wherein the first angle and the second angle are any angles in a range of 70 degrees to 90 degrees.
(((6))) The information processing system according to (((2))),
   wherein the irradiation with the light from the one direction is performed by reducing an intensity of light at an irradiation position by the light from the other direction relative to an intensity of light at the irradiation position by the light from the one direction, and
   the irradiation with the light from the other direction is performed by reducing the intensity of light at the irradiation position by the light from the one direction relative to the intensity of light at the irradiation position by the light from the other direction.
(((7))) The information processing system according to (((6))),
   wherein the reducing of the intensity of light at the irradiation position by the light from the other direction is realized by reducing a luminous intensity of the light from the other direction, and
   the reducing of the intensity of light at the irradiation position by the light from the one direction is realized by reducing a luminous intensity of the light from the one direction.
(((8))) The information processing system according to (((6))),
   wherein the reducing of the intensity of light at the irradiation position by the light from the other direction is realized by restricting the light from the other direction by one member, and
   the reducing of the intensity of light at the irradiation position by the light from the one direction is realized by restricting the light from the one direction by another member.
(((9))) The information processing system according to (((2))),
   wherein the deriving of the expansion and contraction ratio is performed by a combination of any two or more of a first fiber orientation checked by the first image, a second fiber orientation checked by the second image, or a difference between the first fiber orientation and the second fiber orientation.
(((10))) The information processing system according to any one of (((1))) to (((8))),
   wherein a fiber density of the paper is checked from a direction different from the plurality of directions, and
   the deriving of the expansion and contraction ratio is performed based on the fiber orientation and the fiber density.
(((11))) The information processing system according to (((10))),
   wherein the direction different from the plurality of directions is a direction in which light is emitted to an irradiation position of one surface of the paper irradiated with light from one direction and light from another direction, from another surface of the paper, and
   the checking of the fiber density is performed by using an image of the light with which the other surface is irradiated and which is transmitted through the paper.
(((12))) The information processing system according to (((11))),
   wherein the deriving of the expansion and contraction ratio is performed by a combination including the fiber density, and including at least one of a first fiber orientation checked by a first image, a second fiber orientation checked by a second image, or a difference between the first fiber orientation and the second fiber orientation.
(((13))) The information processing system according to any one of (((1))) to (((12))),
   wherein a temperature at which an image formed on the paper is to be fixed is controlled based on the derived expansion and contraction ratio.
(((14))) The information processing system according to (((13))),
   wherein in a case where the expansion and contraction ratio is equal to or higher than a predetermined value, the fixing temperature is set to be lower than a predetermined temperature.
(((15))) The information processing system according to (((13))),
   wherein in a case where the expansion and contraction ratio is equal to or lower than a predetermined value, the fixing temperature is set to be higher than a predetermined temperature.
(((16))) A program causing an information processing system to realize:
   a function of checking a fiber orientation of paper from a plurality of directions;
   a function of deriving an expansion and contraction ratio of the paper based on the checked fiber orientation.

According to (((1))), it is possible to shorten a time taken to grasp the expansion and contraction ratio, as compared with a case where the expansion and contraction ratio of the paper is measured by using a device in the related art.

According to (((2))), a burden of the checking process can be reduced, as compared with a case where the configuration is not adopted in which the checking of the fiber orientation is performed by using the first image in a case where the surface of the paper is irradiated with light in the one direction of the plurality of directions and the second image in a case where the surface of the paper is irradiated with light in the other direction of the plurality of directions.

According to (((3))), accuracy of the expansion and contraction ratio can be improved, as compared with a case where the configuration in which the irradiation of light from the one direction is performed at the first angle with respect to the other direction and the irradiation of light from the other direction is performed at the second angle with respect to the one direction is not adopted.

According to (((4))), an angle according to the paper can be set, as compared with a case where the configuration is not adopted in which the first angle is any angle in a range determined based on the error with respect to the expansion and contraction ratio corresponding to an angle with respect to the other direction, and the second angle is any angle in a range determined based on the error with respect to the expansion and contraction ratio corresponding to an angle with respect to the one direction.

According to (((5))), accuracy of the expansion and contraction ratio can be improved, as compared with a case where the first angle and the second angle are not any angle within a range of 70 degrees to 90 degrees.

According to (((6))), it is possible to suppress blurriness of a shadow, as compared with a case where the configuration is not adopted in which the irradiation of the light from the one direction is performed by reducing the intensity of light at the irradiation position by the light from the other direction than the intensity of light at the irradiation position by the light from the one direction, and the irradiation of the light from the other direction is performed by reducing the intensity of light at the irradiation position by the light from the one direction than the intensity of light at the irradiation position by the light from the other direction.

According to (((7))), a fluctuation in the luminous intensity of the light during the lighting can be suppressed, as compared with a case where the configuration is not adopted in which the reducing of the intensity of light at the irradiation position by the light from the other direction is realized by reducing the luminous intensity of the light from the other direction, and the reducing of the intensity of light at the irradiation position by the light from the one direction is realized by reducing the luminous intensity of the light from the one direction.

According to (((8))), it is possible to reduce a control burden, as compared with a case where the configuration is not adopted in which the reducing of the intensity of light at the irradiation position by the light from the other direction is realized by restricting the light from the other direction by the one member, and the reducing of the intensity of light at the irradiation position by the light from the one direction is realized by restricting the light from the one direction by the other member.

According to (((9))), an error of the expansion and contraction ratio can be suppressed, as compared with a case where the configuration is not adopted in which the expansion and contraction ratio is derived by a combination of any two or more of the first fiber orientation checked by the first image, the second fiber orientation checked by the second image, and the difference between the first fiber orientation and the second fiber orientation.

According to (((10))), the accuracy of the expansion and contraction ratio can be improved as compared with a case where the configuration is not adopted in which the fiber density of the paper is checked from a direction different from the plurality of directions, and the expansion and contraction ratio is derived based on the fiber orientation and the fiber density.

According to (((11))), the accuracy of the expansion and contraction ratio can be improved, as compared with a case where the configuration is not adopted in which the direction different from the plurality of directions is a direction in which light is emitted to the irradiation position of one surface of the paper irradiated with light from one direction and light from another direction, from another surface of the paper, and the checking of the fiber density is performed by using an image of the light with which the other surface is irradiated and which is transmitted through the paper.

According to (((12))), the error of the expansion and contraction ratio can be suppressed, as compared with a case where the configuration is not adopted in which the deriving of the expansion and contraction ratio is performed by a combination including the fiber density, and including at least one of the first fiber orientation checked by the first image, the second fiber orientation checked by the second image, or the difference between the first fiber orientation and the second fiber orientation.

According to (((13))), it is possible to suppress occurrence of curling of the paper, as compared to a case where the configuration is not adopted in which the temperature at which the image formed on the paper is to be fixed is controlled based on the derived expansion and contraction ratio.

According to (((14))), the occurrence of curling of the paper can be suppressed, as compared with a case where the configuration is not adopted in which in a case where the expansion and contraction ratio is equal to or higher than the predetermined value, the fixing temperature is set to be lower than the predetermined temperature.

According to (((15))), the occurrence of curling of the paper can be suppressed, as compared with a case where the configuration is not adopted in which in a case where the expansion and contraction ratio is equal to or lower than the predetermined value, the fixing temperature is set to be higher than the predetermined temperature.

According to (((16))), it is possible to shorten the time taken to grasp the expansion and contraction ratio, as compared with a case where the expansion and contraction ratio of the paper is measured by using the device in the related art.

The foregoing description of the exemplary embodiments of the present invention has been provided for the purposes of illustration and description. It is not intended to be exhaustive or to limit the invention to the precise forms disclosed. Obviously, many modifications and variations will be apparent to practitioners skilled in the art. The embodiments were chosen and described in order to best explain the principles of the invention and its practical applications, thereby enabling others skilled in the art to understand the invention for various embodiments and with the various modifications as are suited to the particular use contemplated. It is intended that the scope of the invention be defined by the following claims and their equivalents.

### Brief Description of the Reference Symbols

1: image forming apparatus
7: media sensor
7a, 7b, 7c: light source
7d: light detector
7e: shutter member
10: paper
10a: one surface
10b: other surface
10c: one side
10d: other side
10e: portion
21b: CPU
27: information processing portion
27a: image acquisition portion
27b: expansion and contraction ratio deriving portion
27c: image correction portion
80: fixing device

## Claims

1. An information processing system comprising:
a processor configured to:
check a fiber orientation indicating an orientation state of fibers in paper from a plurality of directions; and
derive an expansion and contraction ratio of the paper based on the checked fiber orientation.

2. The information processing system according to claim 1,
wherein the checking of the fiber orientation is performed by using a first image in a case where a surface of the paper is irradiated with light from one direction as the plurality of directions and a second image in a case where the surface of the paper is irradiated with light from another direction as the plurality of directions.

3. The information processing system according to claim 2,
wherein the irradiation with the light from the one direction is performed at a first angle with respect to the other direction, and
the irradiation with the light from the other direction is performed at a second angle with respect to the one direction.

4. The information processing system according to claim 3,
wherein the first angle is any angle in a range determined based on an error with respect to the expansion and contraction ratio corresponding to an angle with respect to the other direction, and
the second angle is any angle in a range determined based on an error with respect to the expansion and contraction ratio corresponding to an angle with respect to the one direction.

5. The information processing system according to claim 3,
wherein the first angle and the second angle are any angles in a range of 70 degrees to 90 degrees.

6. The information processing system according to claim 2,
wherein the irradiation with the light from the one direction is performed by reducing an intensity of light at an irradiation position by the light from the other direction relative to an intensity of light at the irradiation position by the light from the one direction, and
the irradiation with the light from the other direction is performed by reducing the intensity of light at the irradiation position by the light from the one direction relative to the intensity of light at the irradiation position by the light from the other direction.

7. The information processing system according to claim 6,
wherein the reducing of the intensity of light at the irradiation position by the light from the other direction is realized by reducing a luminous intensity of the light from the other direction, and
the reducing of the intensity of light at the irradiation position by the light from the one direction is realized by reducing a luminous intensity of the light from the one direction.

8. The information processing system according to claim 6,
wherein the reducing of the intensity of light at the irradiation position by the light from the other direction is realized by restricting the light from the other direction by one member, and
the reducing of the intensity of light at the irradiation position by the light from the one direction is realized by restricting the light from the one direction by another member.

9. The information processing system according to claim 2,
wherein the deriving of the expansion and contraction ratio is performed by a combination of any two or more of a first fiber orientation checked by the first image, a second fiber orientation checked by the second image, or a difference between the first fiber orientation and the second fiber orientation.

10. The information processing system according to any one of claims 1 to 8,
wherein a fiber density of the paper is checked from a direction different from the plurality of directions, and
the deriving of the expansion and contraction ratio is performed based on the fiber orientation and the fiber density.

11. The information processing system according to claim 10,
wherein the direction different from the plurality of directions is a direction in which light is emitted to an irradiation position of one surface of the paper irradiated with light from one direction and light from another direction, from another surface of the paper, and
the checking of the fiber density is performed by using an image of the light with which the other surface is irradiated and which is transmitted through the paper.

12. The information processing system according to claim 11,
wherein the deriving of the expansion and contraction ratio is performed by a combination including the fiber density, and including at least one of a first fiber orientation checked by a first image, a second fiber orientation checked by a second image, or a difference between the first fiber orientation and the second fiber orientation.

13. The information processing system according to any one of claims 1 to 12,
wherein a temperature at which an image formed on the paper is to be fixed is controlled based on the derived expansion and contraction ratio.

14. The information processing system according to claim 13,
wherein in a case where the expansion and contraction ratio is equal to or higher than a predetermined value, the fixing temperature is set to be lower than a predetermined temperature.

15. The information processing system according to claim 13,
wherein in a case where the expansion and contraction ratio is equal to or lower than a predetermined value, the fixing temperature is set to be higher than a predetermined temperature.
